(19) Europäisches Patentamt
European Patent Office
Office européen des brevets

(11) **EP 1 521 839 B1**

(12) **EUROPEAN PATENT SPECIFICATION**

(45) Date of publication and mention
of the grant of the patent:
**08.09.2010 Bulletin 2010/36**

(51) Int Cl.:
*C12N 15/86* (2006.01)     *C12N 5/10* (2006.01)
*A61K 35/76* (2006.01)     *A61K 48/00* (2006.01)

(21) Application number: **03740792.1**

(22) Date of filing: **11.07.2003**

(86) International application number:
**PCT/GB2003/003012**

(87) International publication number:
**WO 2004/007735 (22.01.2004 Gazette 2004/04)**

(54) **METHODS OF MAKING VIRAL PARTICLES HAVING A MODIFIED CELL BINDING ACTIVITY AND USES THEREOF**

METHODE ZUR HERSTELLUNG VON VIRALEN PARTIKELN WELCHE EINE VERÄNDERTE ZELLBINDUNGSAKTIVITÄT HABEN UND DEREN VERWENDUNGEN

METHODES PERMETTANT D'OBTENIR DES PARTICULES PRESENTANT UNE ACTIVITE DE LIAISON CELLULAIRE MODIFIEE ET UTILISATIONS CORRESPONDANTES

(84) Designated Contracting States:
**AT BE BG CH CY CZ DE DK EE ES FI FR GB GR HU IE IT LI LU MC NL PT RO SE SI SK TR**

(30) Priority: **11.07.2002 GB 0216081**

(43) Date of publication of application:
**13.04.2005 Bulletin 2005/15**

(73) Proprietor: **Imperial Innovations Limited
Imperial College
London SW7 2AZ (GB)**

(72) Inventor: **CASIMIR, Colin, Maurice
London N2 9PT (GB)**

(74) Representative: **Wainwright, Jane Helen et al
Potter Clarkson LLP
Park View House
58 The Ropewalk
Nottingham NG1 5DD (GB)**

(56) References cited:
**WO-A-94/20621     WO-A-97/12049**

• **BITTNER ALEXANDRA ET AL: "Specific transduction of HIV-1 envelope expressing cells by retroviral vectors pseudotyped with hybrid CD4/CXCR4 receptors" JOURNAL OF VIROLOGICAL METHODS, vol. 104, no. 1, June 2002 (2002-06), pages 83-92, XP002259019 ISSN: 0166-0934**

• **JIANG AN ET AL: "Cell-type-specific gene transfer into human cells with retroviral vectors that display single-chain antibodies" JOURNAL OF VIROLOGY, vol. 72, no. 12, December 1998 (1998-12), pages 10148-10156, XP002103152 ISSN: 0022-538X**

• **LAVILLETTE DIMITRI ET AL: "Retargeting gene delivery using surface-engineered retroviral vector particles" CURRENT OPINION IN BIOTECHNOLOGY, vol. 12, no. 5, October 2001 (2001-10), pages 461-466, XP002259021 ISSN: 0958-1669**

• **GOLLAN TIMOTHY J ET AL: "Redirecting retroviral tropism by insertion of short, nondisruptive peptide ligands into envelope" JOURNAL OF VIROLOGY, vol. 76, no. 7, April 2002 (2002-04), pages 3558-3563, XP002259022 ISSN: 0022-538X cited in the application**

• **GOLLAN TIMOTHY J ET AL: "Selective targeting and inducible destruction of human cancer cells by retroviruses with envelope proteins bearing short peptide ligands" JOURNAL OF VIROLOGY, vol. 76, no. 7, April 2002 (2002-04), pages 3564-3569, XP002259023 ISSN: 0022-538X cited in the application**

EP 1 521 839 B1

**Description**

The present invention relates to a method for making enveloped viral particles such that one or more peptides on the surface of the virus particle are derived from the packaging cell. By incorporating certain peptides it is possible to target viral particles to specific cell types. Such a system is of use, for example, in gene therapy treatments, wherein the gene therapy is to introduce a bioactive agent which is active in or on a target cell and has a direct or indirect cytotoxic function; or is an enzyme capable of converting a prodrug into a cytotoxic drug.

The development of somatic gene therapy as a treatment for some acquired conditions, such as certain types of cancer, represents one of the most important technical advances in medicine.

However, many problems stand in the way of the realisation of the promise of these techniques.

The ability to deliver genes encoding bioactive agents accurately and efficiently to selected target cell populations in vivo would greatly expand the scope of therapy, but current vectors are not well suited for this task.

Retroviral vectors offer a very high efficiency of chromosomal integration and are therefore well suited to gene therapy strategies, where it is a requirement that the therapeutic gene should be stably transmitted to future progeny of the target cell. Recombinant retroviruses have therefore been used in many clinical gene therapy protocols for ex vivo transduction of cultured T lymphocytes, fibroblasts, keratinocytes, hepatocytes, haemopoietic stem cells and neoplastic cells.

There are very few active clinical trials in which retroviral vectors or vector - producing cells are being used for in vivo transduction of neoplastic cells, by direct inoculation of tumour deposits or by instillation into a cavity (eg bladder, peritoneal or pleural space) whose wall is infiltrated by tumour.

However, for in vivo transduction of lymphocytes, haematopoietic and other stem cells, vascular endothelial cells and disseminated malignancies, it will be necessary to develop retroviral vectors that adhere selectively to a target cell population when administered, especially when administered intravenously. Nontargeted vectors are inadequate since they will adhere predominantly to nontarget cells when introduced into the bloodstream leading to massive vector wastage and increasing the likelihood of undesirable side-effects.

Several approaches to developing retroviral vectors that can target specific cell types have received considerable attention in recent times.

It is possible to modify retroviral particles chemically to try and facilitate their entry into human target cells. Lactose coated β - galactosidase - transducing retroviral particles can specifically bind to the asialoglycoprotein receptor on the human HepG2 cells. However, the process is very inefficient and this form of chemical modification does not seem to offer a broad approach (Neda *et al.,* 1991). Molecular bridges between the target cell and the transducing viral particles have also been tried to no effect (Goud *et al.,* 1998) or at a cost of very low efficiency of transduction (Roux *et al.,* 1989; Etienne - Juan *et al.*, 1992).

An alternative approach is to engineer viral specificity by modifying the viral envelope glycoprotein binding site such that novel polypeptide sequences are displayed which confer a degree of target cell specificity. Such an approach has led to a widening of viral tropism, however, a disadvantage of modifying the viral envelope glycoprotein was a reduction in the efficiency of viral particle formation (titre) (Valsesia - Wittmann *et al.*, 1994).

Alternatively, it is possible to replace the entire retroviral binding site with a new binding domain to confer an entirely new target cell binding activity to the retrovirus (Kasahara *et al.*, 1994; Han *et al.*, 1995; Matano *et al.*, 1995). However, this approach has yet to be successfully repeated (Cosset and Russell, 1996).

Attempts have also been made to add new polypeptide sequences to the viral envelope by genetically modifying the viral genes encoding the surface glycoproteins. In this approach the native retroviral binding domain remains intact and novel target cell specific binding domains are added to the virus. Many polypeptide binding domains, including several single chain antibody fragments and polypeptide growth factors, have now been expressed as N-terminal extensions of the Moloney murine leukaemia virus (MLV) SU glycoproteins (Cosset *et al.*, 1995; Schnierle *et al.*, 1996; Somia *et al.*, 1995; Russell *et al.*, 1993; Marin *et al.*, 1996; Valsesia - Wittmann *et al.*, 1996). However, viral incorporation of such chimeras is usually reduced several-fold compared to wild-type envelopes, presumably reflecting a reduced efficiency of folding and/or oligomerisation (Cossett and Russell, 1996). Hence there appears to be a reduction in viral titre with this approach.

In a recent example of the latter approach, Gollan and Green (2002) J. Virology pp. 3558-3563, attempted to modify MLV tropism by incorporating integrin receptor ligands into the viral envelope. Although the authors concluded that short ligands could be successfully introduced, such an approach is very arduous; in excess of 40 chimeric envelope derivatives were synthesised.

The present invention seeks to provide viral particles which exhibit a modified cell binding activity, that is, a cell binding activity which is different from that of the native virus binding activity.

In a first aspect, the present invention provides a method of making an enveloped viral particle (hereinafter referred to as viral particle) having a modified cell binding activity comprising:

(i) providing a viral packaging cell containing viral nucleic acid encoding a viral particle having a first cell binding activity;

(ii) the viral packaging cell also containing nucleic acid encoding a passenger polypeptide binding moiety wherein the passenger peptide binding moiety is provided at the outer plasma membrane of the cell and is a non-viral peptide other than a chimeric viral peptide made by fusing the native env portion of the viral peptide with a peptide binding moiety.

(iii) the viral packaging cell line also comprising additional nucleic acid which can be expressed to provide a bioactive agent which is active in or on a target cell and wherein the bioactive agent either has a direct or indirect cytotoxic function; or is an enzyme capable of converting a relatively non-toxic pro drug into a cytotoxic drug.

(iv) expressing the viral nucleic acid and nucleic acid encoding the passenger peptide binding moiety so that a viral particle buds from a packaging cell membrane and the passenger peptide binding moiety is provided at the cell membrane such that the passenger peptide binding moiety is incorporated into the viral particle to modify its first cell binding activity, wherein the modified cell binding activity is that the viral particle can interact with one or more different cell types than that of the unmodified viral particle.

Surprisingly, simply expressing a desirable membrane bound peptide within a viral packaging cell allows one to effect incorporation of that peptide into viral particles which bud from a viral packaging cell membrane. Hence, the methods of the invention obviate the need to genetically engineer viral particles to express the peptide as a fusion with one or more proteins at the surface of a viral particle, eg the env protein of retroviruses. If the membrane bound polypeptide was, for example, a ligand that binds specifically to a target cell type, then the viral particle of the invention would specifically target that cell type. Accordingly, the viral particles of the invention also provide a means of delivering a bioactive agent to a target cell.

A further surprising benefit of the methods of the invention is that there appears to be no significant reduction in viral titre. As discussed above, existing methods of producing viral vectors with modified cell binding activity lead to a significant and undesirable reduction in viral titre.

Without wishing to be bound by any particular theory, we believe that the viral particles incorporate 'passenger' peptides into the viral envelope during viral budding.

The methods of the invention have the still further advantage that a single type of viral particle can have a common effect upon many different cell types in a cell type-specific manner: by simply transferring a single type of viral particle into different packaging cell lines that express a single target cell specific polypeptide on a cell surface membrane through which the virus particle buds, it is possible to manufacture a range of viral particles, each having a different cell type specific tropism conferred by the passenger peptide incorporated into its viral envelope. In other words, the invention allows one to select target cell specificity by selecting the viral packaging cell, rather than the existing approaches which involve genetically engineering a different viral particle.

It is preferred that the passenger peptide binding moiety is normally a membrane-bound peptide binding moiety. However, as described below, non-membrane peptide binding moieties can also be expressed as a fusion to polypeptides which incorporate a plasma membrane integration or 'anchor' region.

The packaging cell may be modified to introduce one or more passenger peptide binding moieties to be incorporated into the viral particle. For example, the packaging cell may express two or more different peptides having peptide binding moieties that can interact with the same target cell type, for example a human haematopoietic cell. In this way, it may be possible in increase the efficiency of targeting of the viral particle to a target cell type.

By 'viral packaging cell' we mean a cell in which a viral vector can be expressed and from which viral particles can be produced. Packaging cells may be any animal cells permissive for the virus, or cells modified so as to be permissive for the virus; or the packaging cell construct, for example, with the use of a transformation agent such as calcium phosphate. Cell lines which can be used as packaging cells for retroviruses included rodent cells such as NIH/3T3 cells and other murine cells; a suspension cell line such as Chinese Hamster Ovary cells (CHO) or L929 cells; and the FLY viral packaging cell system outlined in Cosset et al (1995) J Virol 69, 7430-7436.

For example, in the present invention a preferred retroviral packaging cell line is called Phoenix (Grignani et al., 1998; Kinsella and Nolan, 1996), discussed further in Example 2. The Phoenix ecotropic and amphotropic packaging cell lines used in the accompanying examples were kind gifts from Dr Gary Nolan, Stanford University, California, USA. These cell lines are second generation retrovirus-producer lines for the production of helper-free ecotropic and amphotropic retroviruses. The lines are based on the 293T cell line, a human embryonic kidney cell line.

A further preferred retroviral packaging cell line is the FLY viral packaging cell system outlined in Cosset et al (1995) J Virol 69, 7430-7436.

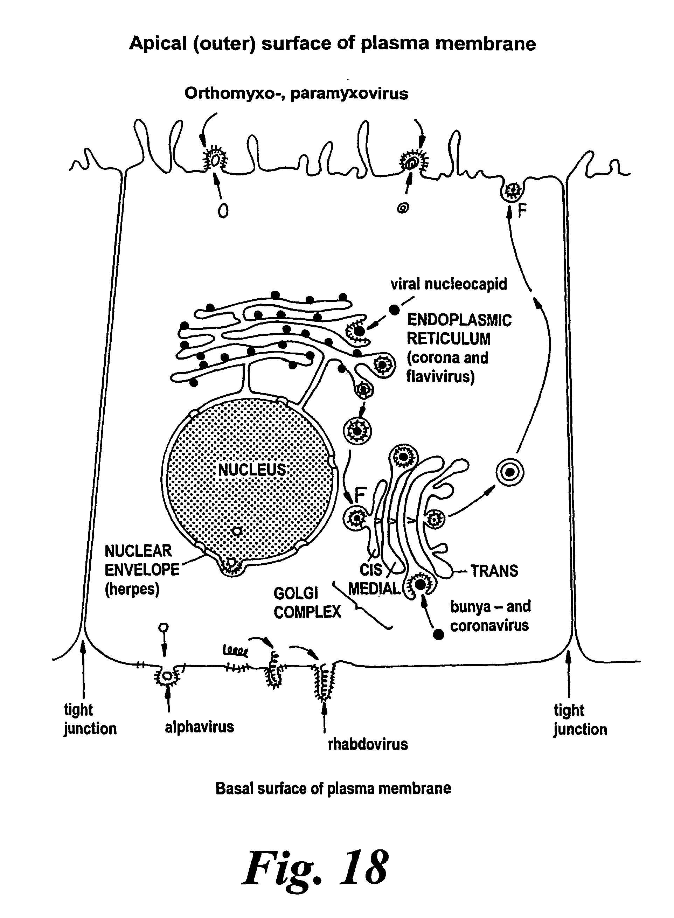

In a preferred embodiment the viral particle is derived from an enveloped - virus, for example retroviruses including rous sarcoma virus, human and bovine T - cell leukaemia virus (HTLV and BLV) and lentiviruses such as human and simian immunodeficiency viruses (HIV and SIV) and Mason - Pfizer monkey virus; foamy virus; herpes viruses (HSV, varicella-zoster, vaccinia); Pox viruses; orthomyxoviruses including influenza; paramyxoviruses including parainfluenza virus, respiratory syncytial virus, Sendai virus, mumps virus and measles virus; corona and flaviviruses; alphaviruses; rhabdoviruses including vesicular stomatitis virus and rabies virus; vaccinia viruses; bunyaviruses, and most RNA viruses, e.g. Rhabdovirus VSV (vesicular stomatitis virus). Also included are those enveloped viruses disclosed in Figure 18 and the following documents: Dimmock and Primrose, (1987), Bodem *et al.*, (1997), Strauss *et al.*, (1995), Griffiths and Rottier (1992), Garoff *et al.*, (1998) and Cadd *et al.*, (1997).

By 'first cell binding activity' we mean the binding activity of the virus which dictates the natural host cell range of the virus. The host range of a virus generally is partially determined by a portion of the virus surface receptor moiety on the surface of the virion. Virus host range is further defined by the unique molecular biology of the infected cell by enhancers/promoters controlling gene expression. Some viruses attach to a specific cell type. The viruses produced by the packaging cell line will also attach to a specific cell type, by use of a natural surface receptor moiety.

By 'binding moiety' we mean a molecule that is available on the surface of the viral particle to bind to a molecule on a target cell. The 'binding moiety' may be a molecule on the virus or virus-like particle modified in such a way that its binding specificity is changed, or it may be a molecule added to, and exposed on the surface of, the viral particle to provide a new binding specificity.

By 'passenger peptide binding moiety' we mean a peptide with a binding moiety expressed by a viral packaging cell that is incorporated into the viral particle during viral budding from a cell membrane.

The term 'peptide' is also intended to embrace polypeptides and proteins.

By 'modified cell binding activity' we mean that the viral particle can interact with one or more different cell types than that of the unmodified viral particle. In this way, the cell binding activity (or 'tropism') of the viral particle may be modified such that the viral particle can bind to a wider range of cell types than the unmodified viral particle, or a narrower range of cell types.

Hence, the method of the invention can be applied to produce viral particles that have a modified cell binding activity such that the viruses can bind to a specific cell type.

By 'packaging cell membrane' we mean any of the membranes from which viral particles bud, including surface (outer) plasma membrane, nuclear envelope, endoplasmic reticulum, and/or golgi complex membrane. Although the majority of enveloped viruses acquire their envelope by budding from the outer plasma membrane, some, such as the herpesviruses, utilise the nuclear membrane listed here (see Figure 18, adapted from Dimmock and Primrose, 1987).

In a preferred embodiment the packaging cell membrane to which the peptide binding moiety is targeted is the outer membrane.

By 'membrane bound polypeptide' we mean a polypeptide that is inserted into the membrane of a cell. Polypeptides can insert into a membrane most commonly because some of the amino acids that comprise the polypeptide are hydrophobic hence may stably integrate into a lipid environment.

Non-membrane-bound polypeptides may be used in the invention if the polypeptides are fused to a region of polypeptides that have a membrane binding region. For example, the membrane - bound stem cell factor that is employed in one embodiment of the invention, and described in more detail in Example 1, may be used as a region of amino acids onto which a non-membrane bound polypeptide may be fused (an 'anchor'). In this case, the hybrid polypeptide would be inserted into the plasma membrane of the cell and, hence, into the viral particles of the invention. Other membrane-bound growth factor trans-membrane regions may also be of use e.g. Flt-3 ligand, M-CSF (Lyman *et al.*, 1995; Gosman *et al.*, 1988)

Another polypeptide that can be used is the influenza haemaglutinin (Hatziioannou et al., 1999; Hatziioannou et al., 1998).

As was discussed above, the method of the invention can be used to modify the cell binding activity of a viral particle. By manipulating the cell binding activity it is possible that the viral particles produced by the method of the invention can bind to specific cell types. Therefore, one application of the method of the invention is to produce viral particles that can deliver one or more bioactive agents to specific cell types.

The viral packaging cell line comprises additional nucleic acid which can be expressed to provide a bioactive agent which is active in or on a target cell.

By 'bioactive agent' we mean a molecule encoded by nucleic acid inserted into the viral genome which may have a biological effect upon a host cell or an organism, preferably a mammal, containing such a cell. For example, the molecule could impart some therapeutic property upon any cell that the viral particle infects, including a direct or indirect cytotoxic effect upon any cell that the viral particle infected. Examples of types of preferred bioactive agents are described below.

By 'infects' we mean that when the viral particle comes into contact with a target cell type its genetic material enters into that cell type. The 'infection' then proceeds according to the type of viral particle. For example, when a retroviral particle infects a target cell type, the retroviral genome is converted from RNA into DNA which is subsequently incorporated into the genome of the target cell. From this point the infection proceeds by the synthesis of new copies of the retroviral nucleic acid from the host genome, the retroviral nucleic acid is subsequently encapsulated with viral protein and bud from the outer plasma membrane of the target host cell.

Viruses that may be suitable for the methods of the invention are discussed in the following papers.

Garoff *et al.,* (1998) discloses that alphaviruses, retroviruses, rhabdoviruses, orthomyxoviruses and paramyxoviruses bud from surface (outer) plasma membrane; coronaviruses bud from membranes between the endoplasmic reticulum and the golgi complex; while hepadnaviruses bud from the endoplasmic reticulum membrane. In particular, retroviruses, rhabdoviruses, orthomyxoviruses and paramyxoviruses are thought to incorporate host cell proteins during budding and may be particularly suitable in the methods of the invention. The disclosure of this document is incorporated herein by reference.

Bodem *et al.*, (1997) describes Human Foamy Virus (HFV), the factors responsible for its preferential budding into cytoplasmic vesicles and its potential as a vector for genetic transfer. The disclosure of this document is incorporated herein by reference.

Griffiths and Rottier, (1992) reviews five groups, herpes-, rota-, corona-, bunya-, and pox- viruses that bud into, or assemble from, different compartments along the biosynthetic pathway. The review focuses on the virally - encoded membrane glycoproteins that are responsible for determining the site of virus assembly. The disclosure of this document is incorporated herein by reference.

By 'nucleic acid' we mean either DNA or RNA. If the viral particle of the invention is a DNA virus, we prefer that the nucleic acid is DNA. If the viral particle of the invention is an RNA virus, such as a retrovirus, then we prefer that the nucleic acid material is RNA.

Methods for inserting nucleic acid into the viral genome are well known in the art and are further described below (see, for example, Sambrook et al., MOLECULAR CLONING: A LABORATORY MANUAL. 2001. 3rd edition).

Although the viral particle of the invention can be derived from any enveloped virus, it is preferred that the viral particle is derived from a retrovirus. Such viruses are commonly used in gene therapies because their viral genome fully and stably integrates into the genome of any infected host cell. Hence any nucleic acids encoding bioactive agents would also be incorporated into the host cell genome.

Conveniently, the viral particle is "replication-defective". By "replication defective" we mean a virus whose genetic material has been manipulated so that it cannot divide or proliferate in the cell it infects on its own. An advantage of such a viral particle is that the virus cannot multiply in the host cell or continue to infect other cells.

In a preferred embodiment the viral particle is derived from the retrovirus murine leukaemia virus (MLV).

Retroviral vectors encoding MLV are widely available to those skilled in the art, such as PINCO (Grignani et al., 1998) or the pBabe vector series (Morgenstern and Land, 1990). Alternatively, the present invention could employ a lentiviral vector such as those based on human immunodeficiency virus (HIV), caprine arthritis encephalitis virus (CAEV) or Visna-Maedi virus (VMV). Further vectors suitable for use in the methods described herein can be readily identified and/or prepared by the skilled person.

In another preferred embodiment the viral particle is derived from the human immunodeficiency virus (HIV). HIV vectors are well known and available to those skilled in the art.

The method of the invention can be employed to modify the cell binding activity of viral particles to deliver a bioactive agent to a target cell type. One such method of targeting a specific cell type is to incorporate a cell growth factor polypeptide on the viral particle. Hence a further embodiment of the invention is that one or more of the passenger peptide binding moieties is a cell growth factor.

By 'cell growth factor' we mean a peptide that acts to induce or repress the growth of a cell to which said peptide binds. Such a growth factor may be, for example, a ligand recognised or recognisable by a specific cell type. In such a case the ligand may act to promote proliferation of that cell type. Examples of such growth factors include, but are not limited to, platelet - derived growth factor (PDGF), fibroblast growth factor (FGF), interleukin 3 (IL - 3), granulocyte-macrophage colony-stimulating factor GM - CSF, epidermal growth factor (EGF), FLT-ligand and stem cell growth factor (SCF), also known as mast cell growth factor, kit ligand factor, or Steel factor. Nucleic acid sequences encoding stem cell factors are described in WO92/00376, eg the Δ28 MGF stem cell factor.

A particularly preferred aspect of the invention is a method of modifying a viral particle so that it can bind to a specific stem cell type. In this way it may be possible to deliver a bioactive agent to a population of stem cells from which a wide range of other cells will differentiate.

Hence a particularly preferred growth factor is membrane - bound stem cell factor.

By 'membrane bound stem cell factor' we mean the peptide binding moiety discussed in Sehgal *et al* (1996). Nucleic acid sequences encoding stem cell factors are described in WO92/00376, eg the Δ28 MGF stem cell factor.

Stem cell factor (SCF) is a ligand that binds to the c - kit receptor protein found on the surface of quiescent stem cells (Hamel and Westphal, 1997). SCF exists in two forms, a longer soluble form and a shorter membrane bound form. The two forms results from differential mRNA splicing, with the soluble form having a proteolytic cleavage site coded by exon 6, while the membrane bound form has no exon 6 and thus no proteolytic cleavage site.

Thus, a preferred embodiment of the invention is a method of producing viral particle comprising a membrane bound stem cell factor within its viral envelope. Hence, the viral particle has a binding moiety that can interact with any cell type that has a molecule on the surface with which the membrane bound stem cell factor can bind. An example of such a molecule is the c - kit receptor. One example of a cell type with the c - kit receptor is pluripotent haematopoietic stem cells (PHSC), also known as haematopoietic stem cells (HSC).

Hence in a preferred embodiment the invention provides a method to target viral particles to haematopoietic stem cells. Such viral particles constitute a means ('vector') of delivering a bioactive agent to said cells.

Of course, using a peptide that acts as a growth factor as a means to modify the cell binding activity of a viral particle is only one such type of passenger peptide binding moiety. The method of the invention can also be used to modify the cell binding activity of a viral particle by adding an antibody, or an antigen binding fragment thereof, in which case the viral particles will take up the cell binding activity of the antibody or fragment thereof. Methods suitable for using antibody-derived cell binding moieties in viral particles are well known to those skilled in the art (Ager *et al.*, 1996; Russell *et al.,* 1993).

Hence a further preferred embodiment of the invention is a method of modifying the cell binding activity of a viral particle as described above wherein the peptide binding moiety is an antibody, or an antigen binding fragment thereof.

In one preferred embodiment the binding moiety has the binding activity of any one of a monoclonal antibody, ScFv (single chain Fv fragment), a dAb (single domain antibody) or a minimal recognition unit of an antibody.

The binding moiety may be a monoclonal antibody. Monoclonal antibodies which will bind to many of these antigens are already known but in any case, with today's techniques in relation to monoclonal antibody technology, antibodies can be prepared to most antigens. The binding moiety may be a part of an antibody (for example a Fab fragment) or a synthetic antibody fragment (for example, ScFv). Suitable monoclonal antibodies to selected antigens may be prepared by known techniques, for example those disclosed in *"*Monoclonal Antibodies: A manual of techniques", H Zola (CRC Press, 1988) and in *"*Monoclonal Hybridoma Antibodies: Techniques and Applications", J G R Hurrell (CRC Press, 1982).

Suitably prepared non-human antibodies can be "humanized" in known ways, for example by inserting the CDR regions of mouse antibodies into the framework of human antibodies.

The variable heavy ($V_H$) and variable light ($V_L$) domains of the antibody are involved in antigen recognition, a fact first recognised by early protease digestion experiments. Further confirmation was found by "humanization" of rodent antibodies. Variable domains of rodent origin may be fused to constant domains of human origin such that the resultant antibody retains the antigenic specificity of the rodent parental antibody (Morrison et al (1984) Proc. Natl. Acad. Sci. USA 81, 6851-6855). That antigenic specificity is conferred by variable domains and is independent of the constant domains is known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); ScFv molecules where the $V_H$ and $V_L$ partner domains are linked via a flexible oligopeptide (Bird et al (1988) Science 242, 423; Huston et al (1988) Proc. Natl. Acad. Sci. USA 85, 5879) and dAbs comprising isolated V domains (Ward et al (1989) Nature 341, 544). A general review of the techniques involved in the synthesis of antibody fragments which retain their specific binding sites is to be found in Winter & Milstein (1991) Nature 349, 293-299.

By "ScFv molecules" we mean molecules wherein the $V_H$ and $V_L$ partner domains are linked via a flexible oligopeptide.

It may be advantageous to use antibody fragments, rather than whole antibodies. Effector functions of whole antibodies, such as complement binding, are removed. ScFv and dAb antibody fragments can be expressed as fusions with other polypeptides.

Minimal recognition units may be derived from the sequence of one or more of the complementary-determining regions (CDR) of the Fv fragment. Whole antibodies, and F(ab')$_2$ fragments are "bivalent". By "bivalent" we mean that the said antibodies and F(ab)$_2$ fragments have two antigen combining sites. In contrast, Fab, Fv, ScFv, dAb fragments and minimal recognition units are monovalent, having only one antigen combining sites.

From the above it can be seen that one application of the invention is a method of producing a viral particle having a cell binding activity conferred by an antibody. In this case, the viral particles produced may bind to any cell type to which the antibody or fragment thereof binds a cell surface antigen. Hence in a further embodiment of the invention, the peptide binding moiety of the viral particles recognises a target cell - specific surface antigen.

It will be appreciated by those skilled in the art that binding moieties which are polypeptides may be conveniently made using recombinant DNA techniques. The binding moiety may be fused to a protein expressed on a membrane of the packaging cell as disclosed above.

Nucleic acid sequences encoding many of the targeting moieties are known, for example those for peptide hormones, growth factors, cytokines and the like and may readily be found by reference to publicly accessible nucleotide sequence databases such as EMBL and GenBank. Once the nucleotide sequence is known it is routine for a person skilled in the art to make DNA encoding the chosen binding moiety using, for example, chemical DNA synthetic techniques or by using the polymerase chain reaction to amplify the required DNA from genomic DNA or from tissue-specific cDNA.

Many cDNAs encoding peptide hormones, growth factors, cytokines and the like, all of which may be useful as binding moieties, are generally available, for example from British Biotechnology Ltd, Oxford, UK.

Examples of cell surface antigens that may be bound by a viral particle produced using the method of the invention are shown in the table below. Also shown are the antibodies that are available that bind these antigens,

**TABLE 1: Cell surface antigens for targeting a) Tumour Associated Antigens**

| Antigen | Antibody | Existing uses |
|---|---|---|
| Carcino-embryonic Antigen | C46 (Amersham) 85A12 (Unipath) | Imaging and therapy of colon/rectum tumours. |
| Placental Alkaline Phosphatase | H 17E2 (ICRF, Travers & Bodmer) | Imaging and therapy of testicular and ovarian cancers. |
| Pan Carcinoma | NR-LU-10 (NeoRx Corporation) | Imaging and therapy of various carcin-omas including small cell lung cancer. |
| Polymorphic Epithelial Mucin (Human milk fat globule) | HMFG1 (Taylor-Papadimitriou, ICRF) | Imaging and therapy of ovarian cancer and pleural effusions. |
| β-human Chorionic Gonadotropin | W14 | Targeting of carboxypeptidase to human xenograft choriocarcinoma in nude mice (Searle et al (1981) Br. J. Cancer 44, 137-144). |
| A carbohydrate on Human Carcinomas | L6 (IgG2a)[1] | Targeting of alkaline phosphatase (Senter et al (1988) PNAS USA 85, 4842-4846. |
| CD20 Antigen on B Lymphoma (normal and neoplastic) | 1F5 (IgG2a)[2] | Targeting of alkaline phosphatase (Senter et al (1988) PNAS USA 85, 4842-4846. |

[1]Hellström et al (1986) Cancer Res. 46, 3917-3923
[2]Clarke et al (1985) Proc. Natl. Acad. Sci. USA 82,1766-1770

Other antigens include alphafoetoprotein, Ca-125 and prostate specific antigen.

**b) Immune Cell Antigens**

| Antigen | Antibody | Existing uses |
|---|---|---|
| Pan T Lymphocyte Surface Antigen (CD3) | OKT-3 (Ortho) | As anti-rejection therapy for kidney transplants. |
| B-lymphocyte Surface Antigen (CD22) | RFB4 (Janossy, Royal Free Hospital) | Immunotoxin therapy of B cell lymphoma. |
| Pan T lymphocyte Surface Antigen (CD5) | H65 (Bodmer and Knowles, ICRF; licensed to Xoma Corp., USA) | Immunotoxin treatment of acute graft versus host disease, rheumatoid arthritis. |

**c) Infectious Agent-Related Antigens**

| Antigen | Antibody | Existing uses |
|---|---|---|
| Mumps virus-related | Anti-mumps polyclonal antibody | Antibody conjugated to diphtheria toxin for treatment of mumps. |
| Hepatitis B Surface Antigen | Anti HBs Ag | Immunotoxin against hepatoma. |

Since it is possible using the method of the invention to produce viral particles that can bind to specific cell types, one application of the invention is to employ the viral particles to introduce into the target cell types a bioactive agent that may be of benefit in the detection, prevention and/or treatment of a disorder associated with the target cell.

Examples of cell types that mediate specific disorders are given in the table above.

Further examples of the types of diseases and disorders that may be treated by virus particles used according to the invention are given below.

A further aspect of the invention is a viral particle having a modified cell binding activity obtainable by the methods of the invention described above, the modified cell binding activity being conferred by a non-viral peptide other than a chimaeric viral polypeptide made by fusing the native env portion of the viral peptide with a peptide binding moiety. Such excluded viral particles include, *inter alia,* those disclosed in WO97/12049 (whose contents are herein incorporated by reference, amongst other things, for the purpose of possible amendment).

By 'other than a chimaeric viral envelope polypeptide' we mean that the modified cell bind activity of the viral particle is not provided by a polypeptide that has been fused to a viral envelope polypeptide encoded by the viral nucleic acid using conventional genetic engineering techniques. Rather, as described above the invention modifies the cell binding activity of a viral particle using a peptide binding moiety expressed by the viral packaging cells.

In a preferred embodiment the specific target cell types that the viral particles bind are mammalian cells, preferably human cells, more preferably quiescent cells. Hence the viral particles of the invention can bind to mammalian quiescent cells.

Advantageously, the specific target cell types that the viral particles bind are human haematopoietic stem cells.

In this application, "quiescent" refers to cells that are unlikely to enter mitosis within the next 24 hours in the absence of appropriate growth stimulus. Preferably, the population of quiescent cells is enriched in haematopoeitic stem cells, for instance by employing the isolation method of Beradi et al (1995) Science 267:104-108 using bone marrow cells. Other quiescent cell types suitable for use in the invention include resting T-lymphocytes, B-lymphocytes and monocytes, stem cells of non-haematopoietic tissues such as liver and muscle, epithelial stem cells in skin, gut, bladder and airways, vascular endothelial cells, quiescent neoplastic cells and germ cells such as sperm progenitors.

In one embodiment, the viral particle includes nucleic acid which encodes a bioactive molecule having a directly or indirectly cytotoxic effect on a target cell. By "directly or indirectly" cytotoxic, we mean that the molecule is itself toxic (for example ricin; tumour necrosis factor; interleukin-2; interferon-gamma; ribonuclease; deoxyribonuclease; Pseudomonas exotoxin A; caspase, etc), or it may be metabolised to form a toxic product, or it may act on something else to form a toxic product. The sequence of ricin cDNA is disclosed in Lamb et al (1985) Eur. J. Biochem. 148, 265-270 incorporated herein by reference.

For example, it would be desirable to target a DNA sequence encoding an enzyme using the virus or virus-like particle of the invention, the enzyme being one that converts a relatively non-toxic prodrug to a toxic drug. The enzyme cytosine deaminase converts 5-fluorocytosine (5FC) to 5-fluorouracil (5FU) (Mullen et al (1922) PNAS 89, 33); the herpes simplex enzyme thymidine kinase sensitises cells to treatment with the antiviral agent ganciclovir (GCV) or aciclovir (Moolten (1986) Cancer Res. 46, 5276; Ezzedine et al (1991) New Biol 3, 608). The cytosine deaminase of any organism, for example *E. coli* or *Saccharomyces cerevisiae,* may be used.

Thus, in a preferred embodiment of the invention, the gene encodes a cytosine deaminase and the patient is concomitantly given 5FC. By "concomitantly", we mean that the 5FC is administered at such a time, in relation to the transformation of the tumour cells, that 5FC is converted into 5FU in the target cells by the cytosine deaminase expressed from the said gene. A dosage of approximately 0.001 to 100.0 mg 5FC/kg body weight/day, preferably 0.1 to 10.0 mg/kg/day is suitable.

Components, such as 5FC, which are converted from a relatively non-toxic form into a cytotoxic form by the action of an enzyme are termed "prodrugs".

Other examples of pro-drug/enzyme combinations include those disclosed by Bagshawe et al (WO 88/07378), namely various alkylating agents and the *Pseudomonas* spp. CPG2 enzyme, and those disclosed by Epenetos & Rowlinson-Busza (WO 91/11201), namely cyanogenic pro-drugs (for example amygdalin) and plant-derived β-glucosidases.

Enzymes that are useful in this embodiment of the invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; β-lactamase useful for converting drugs derivatized with β-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as abzymes, can be used to convert the prodrugs of the invention into free active drugs (see, e.g. R J Massey, Nature, 328, pp. 457-458 (1987)).

Similarly, the prodrugs of this invention include, but are not limited to, the above- listed prodrugs, e.g., phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted by the enzyme of the conjugate into the more active, cytotoxic free drug. Examples of cytotoxic drugs that can be derivatized into a prodrug form for use in this invention include, but are not limited to, etoposide, teniposide, adriamycin, daunomycin, carminomycin, aminopterin, dactinomycin, mitomycins, cis-platinum and cis-platinum analogues, bleomycins, esperamicins (see U.S. Pat. No. 4,675,187), 5-fluorouracil, melphalan and other related nitrogen mustards.

In a further embodiment the gene delivered to the target cell encodes a ribozyme capable of cleaving targeted RNA or DNA. The targeted RNA or DNA to be cleaved may be RNA or DNA which is essential to the function of the cell and cleavage thereof results in cell death or the RNA or DNA to be cleaved may be RNA or DNA which encodes an undesirable protein, for example an oncogene product, and cleavage of this RNA or DNA may prevent the cell from becoming cancerous.

Ribozymes which may be encoded in the genomes of the viruses or virus-like particles herein disclosed are described in Cech and Herschlag "Site-specific cleavage of single stranded DNA" US 5,180,818; Altman *et al* "Cleavage of targeted RNA by RNAse P" US 5,168,053, Cantin *et al* "Ribozyme cleavage of HIV-1 RNA" US 5,149,796; Cech *et al* "RNA ribozyme restriction endoribonucleases and methods", US 5,116,742; Been *et al* "RNA ribozyme polymerases, dephosphorylases, restriction endonucleases and methods, US 5,093,246; and Been *et al* "RNA ribozyme polymerases, dephosphorylases, restriction endoribonucleases and methods; cleaves single-stranded RNA at specific site by transesterification", US 4,987,071, all incorporated herein by reference.

In a still further embodiment the gene delivered to the target cell encodes an antisense RNA.

By "antisense RNA" we mean an RNA molecule which hybridises to, and interferes with the expression from a mRNA molecule encoding a protein or to another RNA molecule within the cell such as pre-mRNA or tRNA or rRNA, or hybridises to, and interferes with the expression from a gene.

Conveniently, a gene expressing an antisense RNA may be constructed by inserting a coding sequence encoding a protein adjacent a promoter in the appropriate orientation such that the RNA complementary to mRNA. Suitably, the antisense RNA blocks expression of undesirable polypeptides such as oncogenes, for example *ras, bcl, src* or tumour suppressor genes such as p53 and *Rb.*

It will be appreciated that it may be sufficient to reduce expression of the undesirable polypeptide rather than abolish the expression.

It will be further appreciated that DNA sequences suitable for expressing as antisense RNA may be readily derived from publicly accessible databases such as GenBank and EMBL.

In a further aspect, the present invention provides compositions comprising viral particles set out above, in combination with a suitable carrier. In this aspect, the present invention provides pharmaceutical compositions suitable for delivering nucleic acid encoding a bioactive agent to a population of stem cells *in vitro*

Alternatively, the composition could be used *in vivo,* to directly deliver the nucleic acid to a patient's own stem cells. In this case, the composition preferably comprises a viral vector incorporating the nucleic acid encoding a bioactive agent and displaying a growth factor on its surface.

By "gene" we mean a nucleic acid coding sequence that may contain introns, or fragment thereof, or cDNA, or fragment thereof.

It is preferred that the passenger binding peptide moiety on the surface of the viral particle and the bioactive agent encoded by the nucleic acid contained with the viral particle are both polypeptides that may be produced by the techniques of genetic engineering.

The nucleotide sequence encoding the bioactive agent is preferably made by an alteration of the viral genome. Similarly, the passenger peptide binding moiety expressed in the packaging cell may be engineered using the methods outlined below.

The nucleotide sequence may be synthesised *de novo* using solid phase phosphoramidite chemistry, but it is more usual for the nucleotide sequence to be constructed from two parts, the first encoding the desirable molecule and the second the virus or virus-like particle. Similarly, the peptide binding moiety expressed in the packaging cell can be fused to a membrane-bound polypeptide region using the methods disclosed here. The two parts may be derived from their respective genes by restriction endonuclease digestion or by other methods known by those skilled in the art such as the polymerase chain reaction.

A variety of methods have been developed to operatively link two nucleotide sequences via complementary cohesive termini. For instance, synthetic linkers containing one or more restriction sites provide a method of joining the two DNA segment together. Each DNA segment, generated by endonuclease restriction digestion, is treated with bacteriophage T4 DNA polymerase of *E. coli* DNA polymerase I, enzymes that remove protruding, 3'-single-stranded termini with their 3'-5'-exonucleolytic activities, and fill in recessed 3'-ends with their polymerizing activities.

The combination of these activities therefore generates blunt-ended DNA segments. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and lifted to an expression vector that has been cleaved with an enzyme that produces termini compatible with those of the DNA segment.

Synthetic linkers containing a variety of restriction endonuclease sites are commercially available from a number of sources including International Biotechnologies Inc, New Haven, CN, USA.

A desirable way to generate the DNA encoding the desirable molecule of the invention and the peptide binding moiety expressed in the packaging cell is to use the polymerase chain reaction as disclosed by Saiki et al (1988) Science 239, 487-491.

In this method each of the DNA molecules are enzymatically amplified using two specific oligonucleotide primers which themselves become incorporated into the amplified DNA. The said specific primers may contain restriction endonuclease recognition sites which may then be used to join the said two DNA molecules using T4 DNA ligase as disclosed.

The present invention also relates to a host cell transformed with a genetic (preferably DNA construct) construct of the present invention. The host cell can be either prokaryotic or eukaryotic. Bacterial cells are preferred prokaryotic host cells and typically are a strain of *E. coli* such as, for example, the *E. coli* strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include yeast and mammalian cells, preferably vertebrate cells such as those from a mouse, rat, monkey or human fibroblastic cell line. Yeast host cells include YPH499, YPH500 and YPH501 which are generally available from Stratagene Cloning Systems, La Jolla, CA 92037, USA. Preferred mammalian host cells include Chinese hamster ovary (CHO) cells available from the ATCC as CCL61, NIH Swiss mouse embryo cells NIH/3T3 available from the ATCC as CRL 1658, and monkey kidney-derived COS-1 cells available from the ATCC as CRL 1650.

Transformation of appropriate cell hosts with a DNA construct of the present invention is accomplished by well known methods that typically depend on the type of vector used. With regard to transformation of prokaryotic host cells, see, for example, Cohen et al (1972) Proc. Natl. Acad. Sci. USA 69, 2110 and Sambrook et al (1989) Molecular Cloning, A Laboratory Manual, Cold Spring Harbor Laboratory, Cold Spring Harbor, NY. Transformation of yeast cells is described in Sherman et al (1986) Methods In Yeast Genetics, A Laboratory Manual, Cold Spring Harbor, NY. The method of Beggs (1978) Nature 275, 104-109 is also useful. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA.

Electroporation is also useful for transforming cells and is well known in the art for transforming yeast cell, bacterial cells and vertebrate cells.

For example, many bacterial species may be transformed by the methods described in Luchansky et al (1988) Mol. Microbiol. 2, 637-646 incorporated herein by reference. The greatest number of transformants is consistently recovered following electroporation of the DNA-cell mixture suspended in 2.5X PEB using 6250V per cm at 25μFD.

Methods for transformation of yeast by electroporation are disclosed in Becker & Guarente (1990) Methods Enzymol. 194, 182.

Successfully transformed cells, ie cells that contain a DNA construct of the present invention, can be identified by well known techniques. For example, cells resulting from the introduction of an expression construct of the present invention can be grown to produce the cytotoxic gene product as defined in the invention. Cells can be harvested and lysed and their DNA content examined for the presence of the DNA using a method such as that described by Southern (1975) J. Mol. Biol. 98, 503 or Berent et al (1985) Biotech. 3, 208. Alternatively, the presence of the protein in the supernatant can be detected using antibodies as described below.

In addition to directly assaying for the presence of recombinant DNA, successful transformation can be confirmed by well known immunological methods when the recombinant DNA is capable of directing the expression of the protein. For example, cells successfully transformed with an expression vector produce proteins displaying appropriate antigenicity. Samples of cells suspected of being transformed are harvested and assayed for the protein using suitable antibodies.

Thus, in addition to the transformed host cells themselves, the present invention also contemplates a culture of those cells, preferably a monoclonal (clonally homogeneous) culture, or a culture derived from a monoclonal culture, in a nutrient medium.

When the genetic construct is a plasmid DNA construct it can be purified. The DNA construct of the invention is purified from the host cell using well known methods.

For example, plasmid vector DNA can be prepared on a large scale from cleaved lysates by banding in a CsCl gradient according to the methods of Clewell & Helinski (1970) Biochemistry 9, 4428-4440 and Clewell (1972) J. Bacteriol. 110, 667-676. Plasmid DNA extracted in this way can be freed from CsCl by dialyse against sterile, pyrogen-free buffer through Visking tubing or by size-exclusion chromatography.

Alternatively, plasmid DNA may be purified from cleared lysates using ionexchange chromatography, for example those supplied by Qiagen. Hydroxyapatite column chromatography may also be used.

The viral particles produced using the method of the present invention can be modified to bind to specific cell types, as described above. One application of this feature of the viral particles is in a method of enriching/purifying target cell types from a larger population of cells.

Hence in a further aspect of the invention, the viral particles of the invention may be used as a means by which an enriched population of a target cell type from a larger population of cells is prepared, wherein: (1) enveloped viral particles of the invention, having a modified binding activity for target cells, are exposed to a population of cells comprising the target cell type to permit binding to the viral particles; (2) enveloped viral particles bound to target cells are then separated from the population of cells; (3) optionally, the enveloped viral particles are subsequently removed from the target cells.

By 'enriching' we mean that the proportion of target cells present in a sample cell after the method outlined above has been employed is substantially greater than the proportion of target cells present in an original population of sample cells.

The viral particles of the invention may be engineered to comprise, for example, a ligand, or a growth factor, or an antibody that may bind with a target cell type. Such a viral particle can then be used as part of an enrichment method as outlined above, which resembles conventional immunoassays familiar to skilled persons.

A further aspect of the invention is a method of enriching the titre of viral particles incorporating a passenger peptide binding moiety from a population of viral particles obtainable by a method as claimed in any preceding claim comprising:

i) providing a support to which the passenger peptide binding moiety binds; and,

ii) exposing the population of viral particles to the support; and, optionally,

iii) isolating the viral particles which bind to the support from the viral particles which do not bind to the support.

By 'enriching the titre' we include the meaning that the titre of the viral particles incorporating a passenger peptide binding moiety is enriched to at least $10^9$ ifu/ml, preferably $10^{10}$ ifu/ml, more preferably $10^{11}$ ifu/ml ('ifu' means 'infection forming units' or 'infectious units'). It is possible to use high titre viral packaging cell systems, for example the FLY system outlined in Cosset et al (1995) J Virol 69, 7430-7436, to increase the titre of the viral particles of the invention.

By 'a population of viral particles' we mean viral particles which have been produced according to the methods of the invention.

The viral particles of the invention may be enriched from a larger population of viral particles, some of which may not have incorporated a passenger peptide binding moiety, using this aspect of the invention. Using a system such as, for example, an immunoaffinity column, a technique well known to those skilled in the art, an antibody to the passenger peptide can bind to those viral particles that have incorporated a passenger peptide. Once bound, viral particles that have not incorporated a passenger peptide binding moiety can be separated from the bound viral particles. Bound viral particles are subsequently released from the column. In this example the immunoaffinity column functions as a support to which the passenger peptide binding moiety binds.

An alternative method of enriching the titre of viral particles of the invention, which is included in this aspect of the invention, is through the use of magnetic microbeads, for example the magnetic beads marketed Miltenyi Biotech (a catalogue and products may be ordered online at www.miltenyibiotec.com). Here, the viral particles of the invention are first bound by an antibody which recognises the passenger peptide. The antibody/viral particle complexes are then bound to magnetic microbeads: this can be via streptavidin/biotin binding between the magnetic microbeads and the antibody, or a Protein G conjugation of the antibody/viral particle complex by the magnetic microbeads. Once the magnetic microbeads/antibody/viral particle complex has been formed, the magnetic microbeads can be separated from the population of unbound viral particles and the viral particles of the invention recovered, as would be appreciated by a person skilled in the art. In this example the microbeads function as a support to which the passenger peptide binding moiety binds

An alternative method of enriching the titre of viral particles of the invention could be the use target cells containing a component, for example the SCF receptor (c-kit) which is bound by the membrane-bound stem cell factor (SCF), to which the passenger peptide binding moiety can bind as a means to enrich viral particles of the invention from a larger population of viral particles. In this example the target cells function as a support to which the passenger peptide binding moiety binds.

Other methods to enrich viral particles that have incorporated a passenger peptide binding moiety will be readily apparent to those skilled in the art. Further examples of supports to which the passenger peptide binding moiety binds will also be readily apparent to those skilled in the art.

A further aspect of the invention is a preparation of viral particles obtainable by any of the previous methods of the invention enriched for viral particles incorporating a passenger peptide binding- moiety, the preparation having a titre of the viral particles of at least $10^5$ ifu/ml.

In order of increasing preference, the preparation of viral particles has a viral titre of at least $10^5$ ifu/ml, $10^6$ ifu/ml, $10^7$ ifu/ml, $10^8$ ifu/ml, $10^9$ ifu/ml, $10^{10}$ ifu/ml, $10^{11}$ ifu/ml, or more.

In a further embodiment of this aspect of the invention the preparation of viral particles further comprises a pharmaceutically acceptable excipient and/or carrier. An example of a suitable excipient is an isotonic buffer such as phosphate buffered saline at pH 7.2, with other suitable excipients being well known to those skilled in the art

A preparation of this aspect of the invention may be prepared by, for example, the method of enriching the titre of viral particles incorporating a passenger peptide binding moiety from a population of viral particles as defined above.

Using the method of the invention it is possible to incorporate a passenger peptide binding moiety that is an antigenic peptide onto the surface of the viral particles of the invention. Hence, a further aspect of the invention provides viral particles of the invention for use as a vaccine.

The vaccine may be administered directly into the patient, into the affected organ or systemically, or applied *ex vivo* to cells derived from the patient or a human cell line which are subsequently administered to the patient, or used *in vitro* to select a subpopulation from immune cells derived from the patient, which are then re-administered to the patient.

Suitably, any viral particles of the invention administered to the patient are sterile and pyrogen free.

The vaccine may be administered without adjuvant. The vaccine may also be administered with an adjuvant such as BCG or alum. Other suitable adjuvants include Aquila's QS21 stimulon (Aquila Biotech, Worcester, MA, USA) which is derived from saponin, mycobacterial extracts and synthetic bacterial cell wall mimics, and proprietory adjuvants such as Ribi's Detox. Quil A, another saponin-derived adjuvant, may also be used (Superfos, Denmark). It is preferred if the vaccine is administered without adjuvant. Other adjuvants such as Freund's may also be useful.

Associated with the above application of viral particles of the invention is a use of the viral particles to present antigenic peptides to T - cells. T-cell antigen recognition requires antigen presenting cells (APCs) to present antigen fragments (peptides) on their cell surface in association with molecules of the major histocompatibility complex (MHC). T cells use their antigen specific T-cell receptors (TCRs) to recognise the antigen fragments presented by the APC. Such recognition acts as a trigger to the immune system to generate a range of responses to eradicate the antigen which has been recognised.

A further aspect of the invention provides a method of delivery of the viral particle which contains a gene encoding a molecule having an indirectly cytotoxic function to a target cell.

Suitably, the indirectly cytotoxic function is an enzyme that converts a prodrug to a toxic drug. With such viral particle, once the viral particle has bound to the target cells, delivered its nucleic acid to the cells, and expressed the indirectly cytotoxic functions, which typically takes a day or so, the pro-drug is administered. The timing between administration of the viral particle and the pro-drug may be optimised in a non-inventive way.

The dosage of the pro-drug will be chosen by the physician according to the usual criteria. The dosage of the viral particle will similarly be chosen according to normal criteria, and in the case of tumour treatment, particularly with reference to the type, stage and location of tumour and the weight of the patient. The duration of treatment will depend in part upon the rapidity and extent of any immune reaction to the virus or virus-like particle.

Some of the viral particles either in themselves, or together with an appropriate pro-drug, are in principle suitable for the destruction of cells in any tumour or other defined class of cells selectively exhibiting a recognisable (surface) entity. Examples of types of cancer that may be treated using the viral particles are cancer of the breast, prostate, colon, rectum, ovary, testicle and brain. The viral particles of the invention are principally intended for human use but could be used for treating other mammals including dogs, cats, cattle, horses, pigs and sheep.

A further aspect of the invention is the use of a viral particle according to the invention in the manufacture of a medicament for the treatment and/or prevention of ovarian cancer.

The viral particles of the invention can target ovarian cancer cells by binding to cell surface antigens present on the cell surface. Once so bound the viral particles can act as a means of delivering a bioactive agent to a target cell. Examples of how such a targeting system can work and examples of bioactive agents are discussed above and further below.

A high proportion of cancer cells, including ovarian cancer cells, express the SCF receptor (c-kit) on the surface of the cell. Other cancer cells expressing the SCF receptor (c-kit) on the surface of the cell include breast, small-cell lung carcinoma, gastrointestinal stromal tumours (GIST), germ cell tumours (eg testicular), acute myeloid leukaemia (AML) and mastocytosis melanoma. Hence it is possible to target cancer cells, including ovarian cancer cells, by incorporating a membrane-bound stem cell factor (SCF) into the viral particle produced by the methods of the invention. The incorporation of a membrane-bound stem cell factor (SCF) into viral particles produced by the methods of the invention is discussed above and in the accompanying examples. Hence a further embodiment of the use of the viral particles in the prevention and/or treatment of cancer, including ovarian cancer, is wherein the virus particles incorporates a membrane-bound stem cell factor (SCF) as a passenger peptide binding moiety.

Recent research has shown that *OPCML*, an opioid binding protein/cell adhesion molecule-like gene, is frequently somatically inactivated in epithelial ovarian cancer (Sellar et al (2003) OPCML at 11q25 is epigenetically inactivated and has tumor-suppressor function in epithelial ovarian cancer. Nat Genet. 34(3):337-43). The authors suggest that *OPCML* is a tumour-suppressor gene, and that loss of OPCML reduces intercellular adhesion and accelerates cell growth at the ovarian surface epithelium, thereby promoting the early steps of ovarian carcinogenesis.

A further aspect of the invention is a pharmaceutical composition comprising the viral particles of the invention and a pharmacutically acceptable carrier. The pharmaceutical formulation is prepared in a form suitable for administration to a patient and is sterile and pyrogen free.

Whilst it is possible for the viral particles to be administered alone, it is preferable to present it as a pharmaceutical formulation, together with one or more acceptable carriers. The carrier(s) must be "acceptable" in the sense of being compatible with the compound and not deleterious to the recipients thereof. Typically, the carriers will be water or saline which will be sterile and pyrogen free.

The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (eg povidone, gelatin, hydroxypropylmethyl cellulose), lubricant, inert diluent, preservative, disintegrant (eg sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

A further embodiment of the invention is the use of a viral particle according to the invention in the manufacture of a medicament for the diagnosis and/or treatment and/or prevention of a disease or a disorder, by virtue of the bioactive agent encoded by the nucleic acid of the viral particle.

In this aspect, the medicament comprising the viral particles is preferably administered by implantation into a patient's bone marrow or is administered by infusion into a patient's blood. In order to allow the viral particles to target the bone marrow when administered by infusion, advantageously, receptors such as integrins can be incorporated on the surface of the viral particles. Alternatively or additionally, the immunogenicity of the viral particles can be reduced by incorporating an immunosuppressive factor such as FAS-ligand on the viral particles surface which can bind to activated T-cell FASreceptors, triggering the T-cells to die by apoptosis.

FAS-ligand expressing allogeneic cell implants have previously been shown to resist immune mediated rejection.

In a further aspect, the present invention provides the use of a viral particle according to the invention in the manufacture of a medicament for the treatment and/or prevention of arthritis.

The viral particles of the invention can target arthritic cells by binding to cell surface antigens present on the cell surface. Once so bound the viral particles can act as a means of delivering a bioactive agent to a target cell. Examples of how such a targeting system can work and examples of bioactive agents are discussed above.

As outlined above, an example of a cell surface antigen found on arthritic cells that may be bound by a viral particle produced by the methods of the invention is the Pan T lymphocyte Surface Antigen (CD5). The cell surface antigen may be bound by incorporating an binding molecule which binds to CD5 into the viral particle produced by the methods of the invention, for example a H65 antibody. Hence an embodiment of this aspect of the invention is wherein the virus particles incorporates a binding molecule which binds to CD5 as a passenger peptide binding moiety.

Alternatively, other binding molecules, for example other antibodies, which bind CD5 can be produced using routine methods, as will be appreciated by a person skilled in the art, and can be incorporated into viral particles produced by the methods of the invention. The incorporation of antibodies into viral particles produced by the methods of the invention is discussed above.

In addition to this, it is possible to target non-arthritic cells which can act as a link between the lymphocytes that attack the joint and the cells of the joint itself. An example of such a non-arthritic cell type is mast cells, as discussed in Lee DM, Friend DS, Gurish MF, Benoist C, Mathis D, Brenner MB. Mast cells: a cellular link between autoantibodies and inflammatory arthritis. Science. 2002 Sep 6;297(5587):1689-92. The paper suggests that if the population of activated mast cells is eradicated then the inflammatory response and the T cells that attack the joint also subside.

SCF is a major regulator of the growth of mast cells which are, therefore, abundant expressers of the SCF receptor (c-kit). Hence it is possible to target mast cells by incorporating a membrane-bound stem cell factor (SCF) into the viral particle produced by the methods of the invention. The incorporation of a membrane-bound stem cell factor (SCF) into viral particles produced by the methods of the invention is discussed above and in the accompanying examples. Hence a further embodiment of this aspect of the invention is wherein the virus particles incorporates a membrane-bound stem cell factor (SCF) as a passenger peptide binding moiety.

In a further aspect, the present invention provides a method of transforming a population of quiescent cells with nucleic acid encoding a bioactive polypeptide so that the nucleic acid is incorporated into the genome of the cells, the method comprising exposing the cells to viral particles as described above, wherein the surface bound growth factor induces the cells to divide, so that the nucleic acid encoding the bioactive polypeptide for treating, preventing and/or diagnosis of a disease or disorder is incorporated into the genome of the cells.

In this aspect, preferably the quiescent cells are a population of bone marrow cells, optionally enriched for haematopoeitic stem cells.

In a further aspect, the viral particles of the invention can be used for gene transfer. By 'gene transfer' we mean that the viral particles of the invention can be used to transfer nucleic acid encoding a bioactive agent to the genome of a target cell.

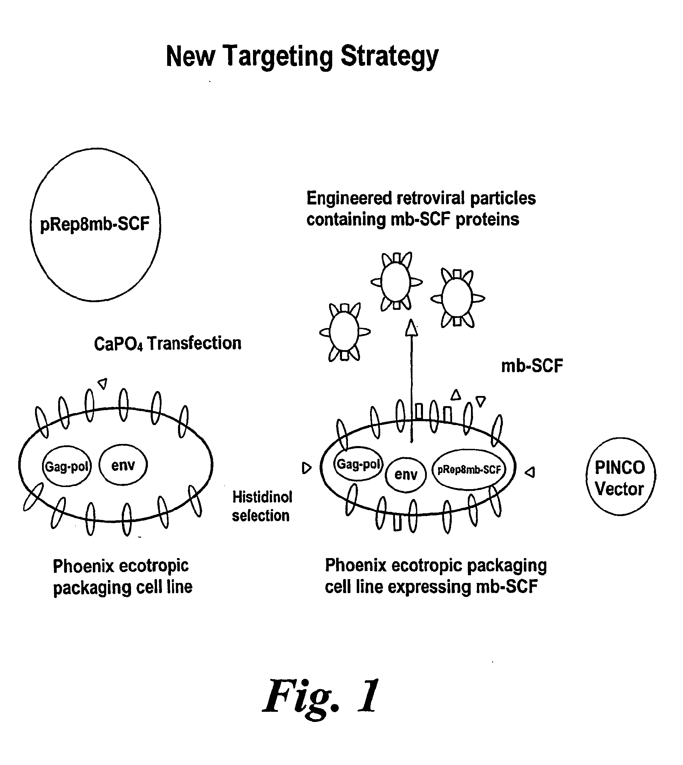

Embodiments of the invention and related technology will now be described in more detail by reference to the following non-limiting Figures and Examples, in which: Figure 1 Strategy for expression of biologically active growth factor on retroviral particle surface. The vectors shown and the methods employed are described further in the description.

Figure 2 Schematic diagram of stem cell factor (SCF) cDNA. The positions of the forward (SCF MF) and reverse primer (SCF MR) used to amplify the SCF cDNA are shown. Primer sequences are described in the description.

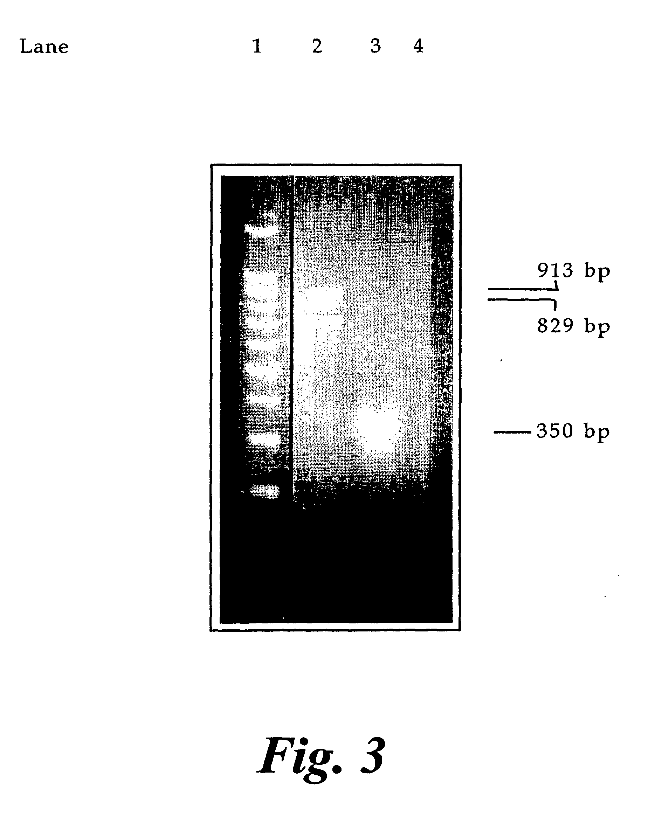

Figure 3 Agarose gel electrophoresis of RT-PCR product from L88. 5 RNA, amplified with SCF primers. L88.5 is a human cell line expressing soluble and membrane-bound forms of the stem cell factor (SCF). Lane 1 - 100bp molecular weight marker; Lane 2-RT-PCR analysis of RNA extracted from L88. 5 cells using SCF MF and MR primers. Three distinct bands were observed, a 913bp fragment indicative of full length SCF encoding the soluble isoform; an 829bp fragment encoding membrane-bound SCF (mb-SCF) ; and an unidentified fragment between 700bp and 800bp, a PCR artefact or possibly another splice variant of the SCF. The 829bp (mb-SCF) fragment was cloned into a PCR cloning vector; Lane 3 - Control PCR reaction from cDNA of L88.5, amplifying the GAPDH gene product of 350bp using GAPDH specific primers; Lane 4-RT-PCR products from reverse transcribed cDNA without the reverse transcriptase as template.

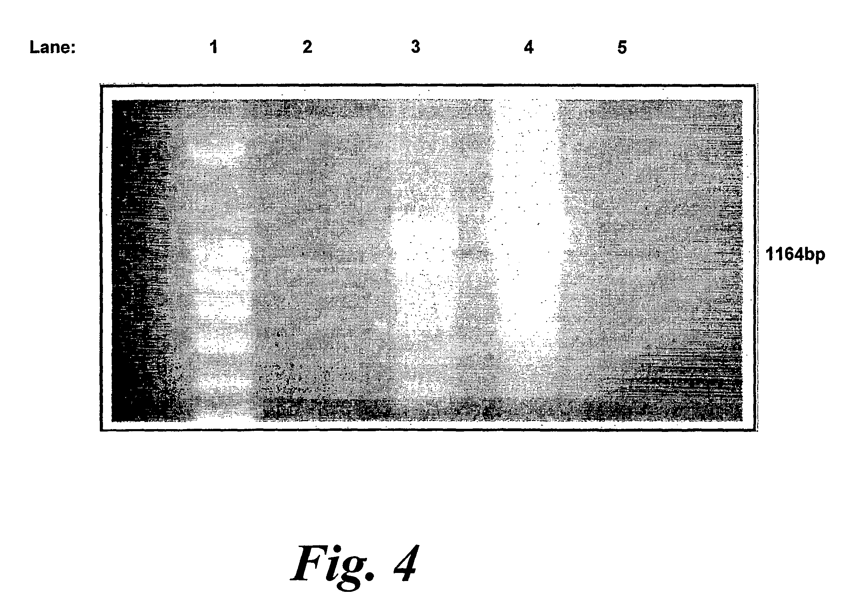

Figure 4 PCR detection of recombinant plasmids in engineered packaging cells. The recombinant plasmid, pRep8mb-SCF, was transfected by calcium phosphate precipitation into the Phoenix ecotropic cell line. Stable clones obtained were propogated and trypsined when cells were about 90% confluent. Two microlitres of a cell suspension (from clone 3) containing these cells were subjected to PCR analysis using PrepF and EBVR primers to detect the presence of the plasmids. Lane 1 - 100bp molecular weight marker; Lane 2 - untransfected ecotropic packaging cells; Lane 3 - Two microlitres of Phoenix ecotropic cell suspension, transfected with pRep8mb - SCF; Lane 4 pRep8mb - SCF control DNA as template for the PCR; Lane 5 - negative control, ie water as template DNA. The expected amplified product of 1164bp was observed in lanes 3 and 4.

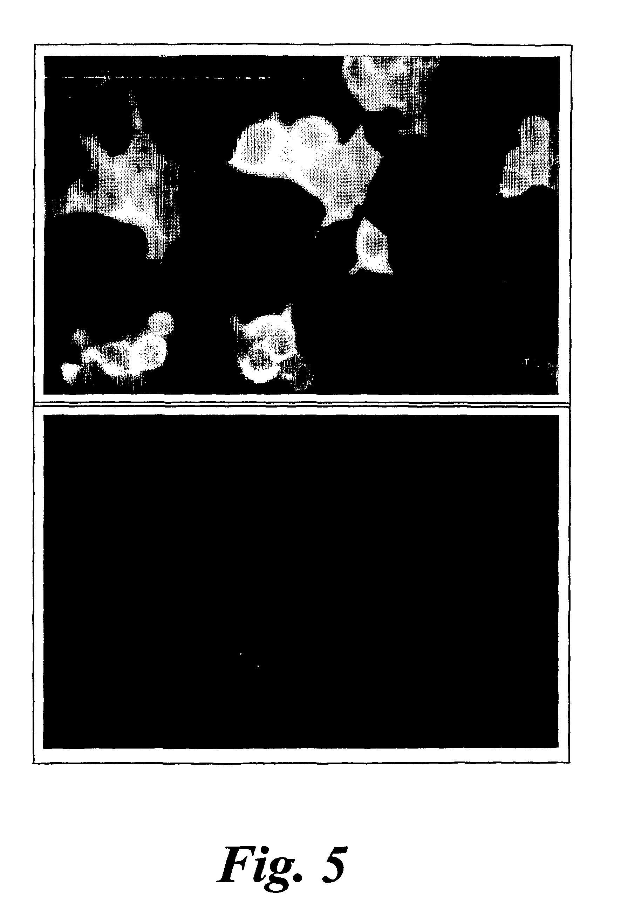

**Figure 5** Immunofluorescence assay of Phoenix ecotropic packaging cell line transfected with pRepmb - SCF. Top panel - transfected cells were grown on glass cover slips and fixed with methanol. The cover slips were stained with anti - SCF antibody, washed twice with PBS and stained with a fluorescent labelled secondary antibody. Cover slips were mounted onto glass slides and viewed under a UV microscope (mag 400X). The figure demonstrates intense surface staining. Bottom panel - negative controls of untransfected cells. These cells were not fluorescent. The exposure time required to capture the negative images (cells) had to be significantly increased.

**Figure 6** Flow cytometry analysis of retroviral packaging cells. Panel A - Unmodified ecotropic retroviral packaging cells. Panel B - Clone 3 packaging cells expressing high levels of mb - SCF proteins. Panel C - retroviral packaging cells expressing CD59.

**Figure 7** Comparison of retroviral titres. Retroviral titres from 3 experiments were determined on NIH 3T3 cells. The mb - SCF modified packaging cells showed a slight but statistically insignificant decrease in titre (paired t test, p=0.5).

**Figure 8** Western blot analysis of retroviral particles. Top panel - retroviral particles expressing the SU portion of the envelope protein. Middle panel - retroviral particles expressing mb - SCF. Bottom panel - retroviral particles expressing CD59, a naturally occurring protein on the packaging cell. Lane assignments; Lane 1 - retroviral particles obtained from stable mb - SCF producing ecotropic cells; Lane 2 - unmodified ecotropic retroviral particles; Lane 3 - retroviral particles obtained transiently from mb - SCF ecotropic packaging cells.

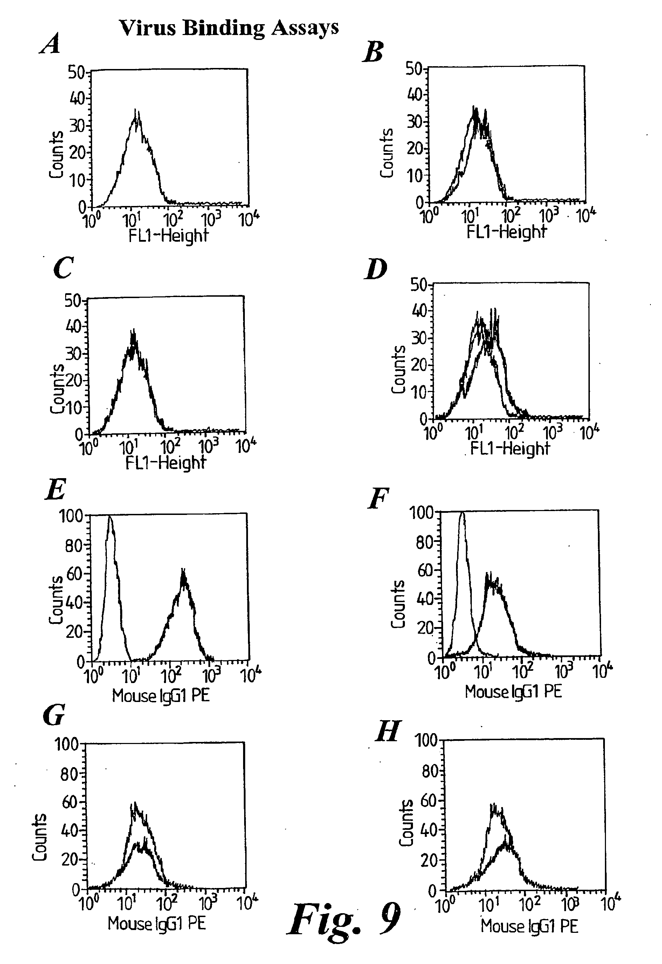

**Figure 9** Retroviral binding assays. Retrovirus was incubated with target cells and the cells stained for bound virus using anti - retroviral envelope monoclonal antibody. Panel B - D show the virus binding data (green trace) superimposed on the control spectrum (grey) from Panel A. Panels G and H show the virus binding data as the heavier black trace superimposed on the control (grey). A) Mo7e cells alone; B) Mo7e + Amphotropic Virus; C) Mo7e +Ecotropic Virus; D) Mo7e + SCF - Ecotropic Virus; Panels E and F. Staining of Mo7e cells with antibody to c - *kit* (black trace) compared to an isotype control (grey trace) before (E) and (F) overnight incubation with 100ng/ml SCF. G) Mo7e cells from panel F + Ecotropic virus; H) Mo7e cells from panel F +Mo7e+SCF - Ecotropic Virus

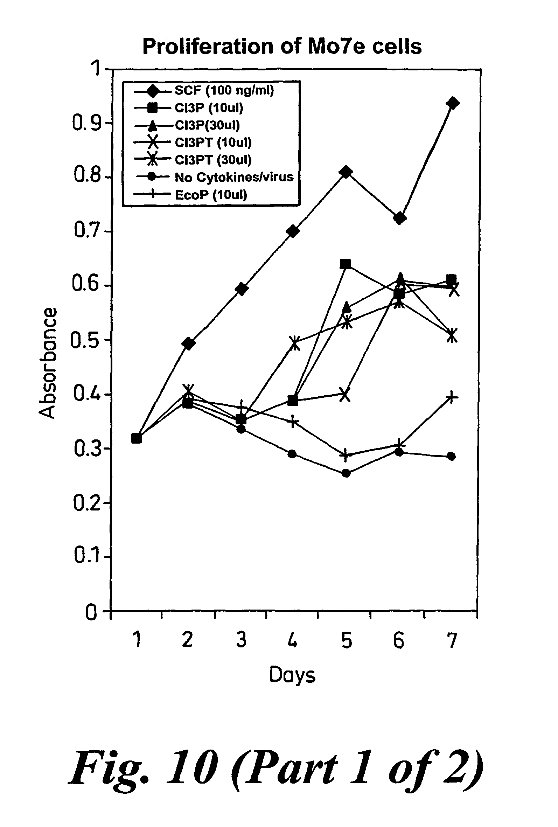

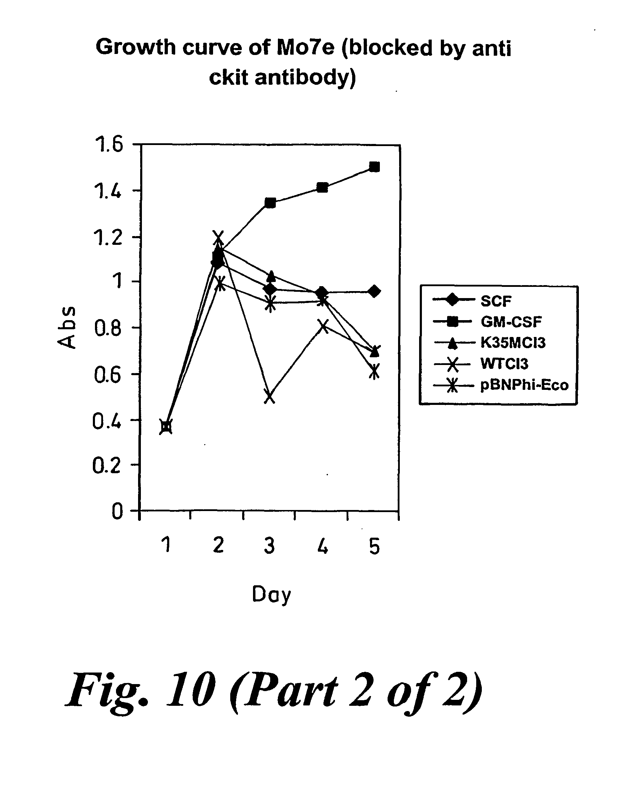

**Figure 10** Proliferation assays using retroviral particles incorporating surface SCF.

**Figure 11** Transduction efficiency of SCF - modified ecotropic retrovirus on human kit positive Mo7e cells.

**Figure 12** Transduction efficiency of SCF - modified ecotropic retrovirus on human kit positive TF - 1 cells.

**Figure 13** Transduction efficiency of SCF - modified ecotropic retrovirus on human kit positive LAMA cells.

**Figure 14** Transduction efficiency of SCF - modified ecotropic retrovirus on human kit negative U937 cells.

**Figure 15** Relative transduction efficiency of SCF - modified ecotropic virus on primary human haematopoietic progenitor cells.

**Figure 16** Relative transduction efficiency of SCF - modified ecotropic virus on mouse bone marrow cells.

**Figure 17** Relative targeted transduction efficiencies of human bone marrow derived CFU - GM

**Figure 18** Schematic diagram showing sites of budding of various enveloped viruses.

**Example 1: Cloning of the human membrane bound SCF cDNA**

This example demonstrates a method for cloning a human gene encoding a membrane bound polypeptide. Stem cell factor (SCF) is a ligand that binds to the c - kit receptor protein found on the surface of quiescent stem cells (Hamel and Westphal, 1997). SCF exists in two forms, a longer soluble form and a shorter membrane bound form. The two forms results from differential mRNA splicing, with the soluble form having a proteolytic cleavage site coded by exon 6, while the membrane bound form has no exon 6 and thus no proteolytic cleavage site (Hamel and Westphal, 1997). We wished to express the membrane bound SCF on the surface of a packaging cell line as a first step to inserting the ligand into the envelope of a viral particle.

*1. A SCF-expressing human cell line*

The human stromal cell line, L88.5, a cell line expressing both the membrane bound form and the soluble form of stem cell factor (SCF) is derived from simian virus 40 (SV40) -transformed human stroma grown from the bone marrow of a haematologically normal patient (Thalmeier *et al.*, 1994). It expresses both isoforms of SCF (the soluble isoform at a higher ratio then the membrane-bound isoform), and was used as the source of membrane-bound SCF (mb-SCF) for cloning. It was routinely maintained in Dulbecco's Modified Eagle's Medium (DMEM) supplemented with 10% heat inactivated Fetal Calf Serum (FCS; Gibco). L88.5 was a kind gift from Dr Karin Thalmeier (Germany).

Cell lines were grown at 37°C in humidified 5%$CO_2$ in air in the incubator (New Brunswick Scientific, UK). Cells grown in suspension were maintained at a density of 2-5 x $10^5$ cells/ml. Adherent cells were passaged at 70-80% confluence: cells were detached from the tissue culture flask on incubation with trypsin-EDTA at 37°C, 5% $CO_2$ in air for 2 minutes, centrifuged at 1200 rpm for 5 minutes and resuspended in the appropriate growth medium.

*2. Isolation of total RNA from human cell line L88.5*

Total RNA was extracted from human cell line L88.5 cells when the cells were sub confluent. Solutions used in RNA manipulation were treated with the RNAase inhibitor diethyl pyrocarbonate (DEPC). Solutions containing 0.1% DEPC were left at room temperature for 12-16 hours Glassware was soaked in DEPC-ddH$_2$O for 2 h, rinsed several times with ddH$_2$O, and autoclaved. DNAse and RNAse free (manufacturer certified) tips and plastic ware were used for all RNA preparation and never reused. Electrophoresis tanks were cleaned with detergent, rinsed in ddH$_2$O, dried with ethanol, soaked in 3% $H_2O_2$ and rinsed thoroughly in DEPC-treated ddH$_2$O. Gloves were worn at all times. RNA extraction was performed using dedicated pipettes. Total RNA was extracted from approximately 5x$10^6$ cells under RNAase-free conditions. Briefly, cells were directly lysed in 0.2ml RNAzol™ B (Biogenesis Limited, Bournemouth, U.K.) per $10^6$ cells. RNA was extracted by the addition of 10% v/v chloroform. Samples were shaken vigorously for 15 seconds, incubated on ice for 5 minutes and centrifuged at 13000 rpm, at 4°C for 15 minutes. The colourless aqueous upper phase containing RNA was removed and RNA was precipitated by the addition of an equal volume of isopropanol, incubation at 4°C for 15 min and centrifugation at 13000rpm, 4°C for 15minutes The RNA pellet was washed in 75% ethanol, and re-pelleted by centrifugation at 7500rpm at 4°C for 15 minutes. The pellet was air dried for 10-15 minutes, and dissolved in sterile DEPC-treated ddH$_2$O. The concentration and purity of total RNA was quantified by optical density (OD) measurement at wavelengths of 260nm and 280nm. An OD of 1 unit (U) corresponds to approximately 40$\mu$g/ml of RNA. OD$_{260}$/$_{280}$ ratios of 1.8 and 2.0 indicate high purities of DNA and RNA respectively. The integrity of extracted RNA was determined by spectrophotometry and agarose gel electrophoresis. The A$_{260}$/$_{280}$ ratio was consistently found to be 2.0 from three different experiments, and all the three species of RNA (28S, 18S and 5S) were detectable by 0.8 % agarose gel electrophoresis.

*3. RT_PCR amplification of the membrane bound - SCF mRNA*

First strand cDNA was then made from the total RNA preparation described above. One $\mu$g of total RNA was used as a template to produce cDNA. A reverse transcription reaction consisting of 1 $\mu$g RNA, 10 mM of dNTP stock, 100 pmol/$\mu$l Random hexameres, 5X RT (Ist strand) buffer, 0.005M DTT and 2U MMLV reverse transcriptase (Gibco, UK) was incubated at 37 °C for 1 hour. The reaction was subsequently heated to 95 °C for 5 min to denature the RT enzyme. The reaction product was held on ice until used or stored long-term at -20 °C.

The cDNA derived from the L88.5 cell line was used as template DNA to clone the membrane-bound SCF by PCR using SCF primers MF and MR.

Figure 1 is a schematic representation of the position of the primers used. The primer sequences used are:

| | |
|---|---|
| SCF MF | ACACCACTGTTTGTGCTGGATC |
| SCF MR | AACTGTTACCAGCCAATGTACG |

The amplification reaction consisted of 2 $\mu$l cDNA, 1$\mu$M SCF forward and reverse primers 200$\mu$M dATP, dCTP, dGTP, dTTP (Sigma), 1x reaction buffer containing 1mM Tris-HCl (pH 9.0 at 25°C), 5mM KCl, 0.01% Triton®X-100), 1.5mM MgCl$_2$, (Sigma) and 1.25U *Taq* DNA polymerase (Sigma) in a total reaction volume of 50$\mu$l. Hot start PCR was carried out by adding the Taq polymerase enzyme after the first 10-15 seconds of the initial denaturing step at 94°C. The annealing temperature was optimised to 60°C for 1 minute and the extension temperature to 72°C for 1 minute. Thirty four cycles were carried out with the final cycle having an increased extension time of 10 minutes. PCR products were electrophoretically analysed in a 2% agarose gel containing 0.5$\mu$g/ml ethidium bromide. DNA was visualised by illumination with shortwave UV radiation (300nm) on a Foto UV 300 series transilluminator (Fotodyne Incorporated), and photographed on Polaroid 667 film (Polaroid U.K. Limited).

Three distinct bands were observed on a 2% agarose gel in three different experiments, including fragments with sizes of 913bp (giving rise to the soluble SCF), 829bp (the membrane-bound-SCF) and an unidentified fragment of about 700bp. As a positive control for the RT-PCR, GAPDH 5' and 3' primers were used, which gave the predicted size fragment of 350bp. The GAPDH primer sequences are shown below.

| GAPDH 5' | GTGAAGGTCGGAGTCAACG |
|----------|---------------------|
| GAPDH3'  | GAGATGATGACCCTTTTGGC |

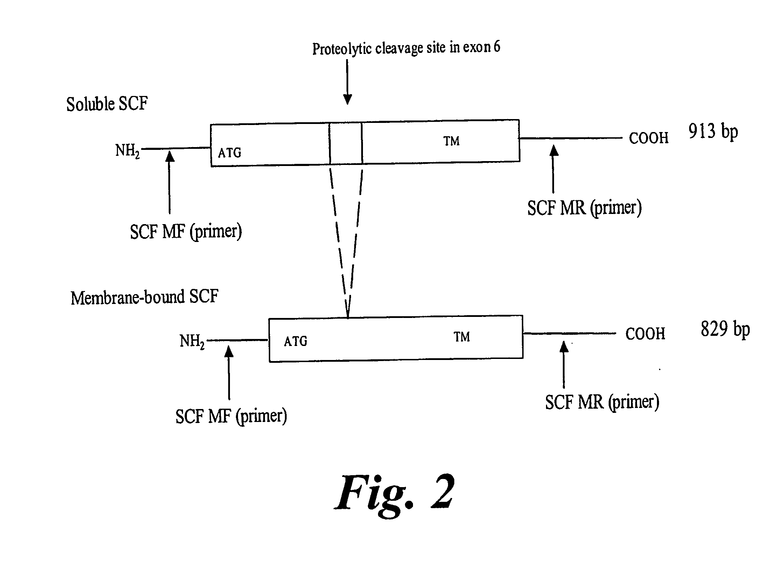

Negative controls were performed by substituting template DNA with water and by omitting the reverse transcriptase enzyme in the RT reaction, using the product as template for the PCR reaction. The results of the RT-PCR reaction are shown in Figure 2.

*4. Cloning of the mb- - SCF cDNA*

PCR-amplified SCF fragments were separated by agarose gel electrophoresis (as described above) and visualised by illumination under shortwave UV radiation (300nm). The 829 bp fragment corresponding to the membrane-bound SCF was excised with a scalpel. The mb-SCF DNA fragment was gel purified using the Advantage™ PCR-Pure Kit (Clontech Laboratories - distributed by Cambridge Bioscience, Cambridge, U.K.) according to the manufacturer's instructions. The supernatant containing the purified mb-SCF cDNA was transferred to a fresh tube and the yield and purity measured by spectrophotometry. Solutions of purified DNA fragments were stored at -20°C.

Ligation of the gel purified mb-SCF fragment DNA with linearised pCR2.1 cloning vector was performed using a 1:3 molar ratio of linearised pCR2.1 vector (50ng): gel purified mb-SCF cDNA fragment (insert DNA). Vector and insert DNA were incubated at 14°C for 14-16 h in the presence of 1x ligase reaction buffer (Clontech, UK): 30mM Tris-HCl pH 7.8, 10 mM MgCl$_2$, 10mM DTT, 0.5mM ATP) and 2U T4 DNA ligase (Promega) in a total volume of 10μl. Positive and negative control ligation reactions comprising manufacturers control plasmid were performed in parallel.

The pCR2.1 cloning plasmid was obtained from Invitrogen (Netherlands) and was used with the TA cloning kit (Invitrogen) for all PCR cloning. The TA Cloning Kit Dual Promoter with pCR II (vector) provided a quick, one-step cloning strategy for the direct insertion of a PCR (or RT-PCR) product into a plasmid vector. The principle of TA cloning is based on the fact that Taq polymerase has a non-template dependent activity that adds a single deoxyadenosine (A) to the 3' ends of PCR products. The linearised plasmid vector (pCR2.1) supplied in the kit has single 3' deoxythymidine (T) residues. This allows PCR inserts to ligate efficiently with the vector

The above ligation reaction was transformed into competent INV Fα strain of *E. coli* (Invitrogen). Frozen competent E.coli cells were thawed on ice. Three μl of the ligation mixture was added to one vial of competent cells and incubated on ice for 30 minutes. Cells were subjected to heat shock at 42°C for 30 seconds and immediately placed on ice for 2 minutes. 250 μl of SOC medium was then added, and the transformation mixture incubated at 37°C for 60 minutes with shaking at 225rpm. The total volume was plated onto 15% Luria Bertani (LB) agar plates containing 100 μg/ml ampicillin and the plates were incubated at 37°C for 14-16 hours. Positive and negative control transformation reactions containing closed circular plasmid DNA and linear DNA or ddH$_2$0 were performed in parallel. Transformation efficiency was calculated by performing a test transformation with closed circular plasmid DNA. The number of colonies produced using 1, 10 and 10ng DNA provides an estimation of colony forming units/μg.

Screening bacterial colonies for recombinant transformants on the basis of β-galactosidase activity is possible if the insert DNA is cloned into an appropriate *lacZ*-peptide-coding vector and transformed in the INV Fα strain of *E.coli*. Appropriate vectors carry a fragment of the *E.coli lac* operon, coding for the amino-terminal fragment of the β-galactosidase enzyme. The INV Fα strain carries a *lac l*$^q$*Z*ΔM15 mutation complemented by intra-allelic-complementation in the presence of the *lacZ*-peptide. Complementation is indicated by the formation of blue colonies in the presence of the β-galactosidase substrate 5-bromo-4-chloro-3-indolyl-β-D-galacto-pyranoside (X-gal). DNA cloned into a multiple cloning site within the β-galactosidase coding sequence of the pCR2.1 cloning vector causes insertional inactivation, resulting in loss of β-galactosidase activity as indicated by the production of white colonies. Briefly, LB agar plates containing 100 μg/ml ampicillin (amp) were pre-coated with 40μl of 20mg/ml X-gal (Boehringer Mannheim) and 4μl of 200mg/ml (800μg) IPTG and allowed to dry for 2-3 hours at 37°C. One hundred μl of the above transformation mix was plated and incubated for 12-16 hours. at 37°C. The resulting white colonies containing the mb-SCF cDNA were screened by restriction digest analysis and PCR using SCF primers MF and MR as above, resulting in an amplification fragment of 829 bp.

The orientation of the fragments in the pCR2.1 vector was also initially determined by PCR using M13-specific forward primers (on the flanking pCR2.1 sites) and SCF-specific primers (MR) (data not shown) and was confirmed by sequencing (Advanced Biotechnology Centre, ICSM Charing Cross Campus) (sequence not shown). The initial clone selected for sequencing revealed a Taq induced error (sequence not shown). Three more clones were then selected and sequenced. Clone 2 had the correct sequence of mb-SCF and was selected for sub-cloning experiments.

*5. Cloning of the mb-SCF cDNA into a mammalian expression vector* The pCR2.1mb-SCF clones were sequenced at The Advanced Biotechnology Centre, Charring Cross Hospital. One clone containing the correct mb-SCF sequence and orientation (clone 2) was used to further sub-clone the mb-SCF insert into the pREP8 expression plasmid. The pREP 8 mammalian expression plasmid was obtained from Invitrogen. This plasmid was designed for high level, constitutive expression of DNA products utilising an RSV promoter. It has a histidinol resistance gene as a selectable marker for the expression of the desired cloned gene. This expression plasmid also contains the Epstein Barr Virus (EBV) Origin of Replication (oriP) and Epstein Barr Nuclear Antigen-1 (EBNA-1) genes enabling the plasmid to replicate as an episome.

Plasmid DNA from pCRII.1mb-SCF clone 2 and pREP8 bacterial cell lines was prepared by growing the bacterial strains in liquid culture. A sterile culture of medium containing the 100 $\mu$g/ml amp was inoculated with bacteria picked from a single, freshly grown colony and incubated for 12-16 hours. at 37°C with shaking at 220 cycles/minutes. Subsequent purification of plasmid DNA from the bacterial culture was performed using the Qiagen MinipREPs™ DNA Purification System (Qiagen), following the manufacturer's instructions. The protocol involved isolation of plasmid DNA by alkaline lysis and subsequent purification by binding to a silica-based matrix. Briefly, 5 ml of bacterial culture was grown as described above and plasmid DNA was extracted according to manufacturer's instruction.

The recombinant pCRII.1mb-SCF clone 2 and pREP8 were both digested with Not I and Hind III restriction enzymes. Digestion of plasmid DNA was carried out by incubation at 37°C for 14 hours in the manufacturer's 1x digestion buffer in the presence of 1-2 U of restriction enzyme. Digestion was confirmed by agarose gel electrophoresis. The mb-SCF cDNA insert DNA in pCRII.1mb-SCF clone 2 was isolated by electrophoresis of the restriction enzyme digestion reaction on an agarose gel and recovery of the insert DNA from the agarose gel as described above. The recovered mb-SCF cDNA was subsequently ligated into the linearised pREP8 vector using the same approach as described above. The ligation mixture was then transformed into transformation competent *E. coli* strain INV-F cells as described above. The resulting recombinant clones, designated pREP8mb-SCF clones, were screened by PCR and mini pREPs to identify a recombinant clone containing a single copy of the mb - SCF cDNA inserted into the pREP8 vector in the correct orientation. One of these clones, termed pREP8mb-SCF, was then selected and used further.

**Example 2: Expression of the mb - SCF cDNA in retroviral packaging cells.**

A cDNA clone coding for the human mb - SCF polypeptide was isolated and cloned into a mammalian expression vector as described above. We wished to then express the mb - SCF on the surface of a packaging cell line as a next step to inserting the polypeptide into the envelope of a viral particle.

*1. Transformation of a viral packaging cell line with pREP8mb-SCF*

Clone pREP8mb-SCF was selected for transformation into a viral packaging cell line. This clone has a cDNA encoding the mb - SCF polypeptide inserted into a mammalian expression vector. As a first step, plasmid DNA was extracted from the clone using a large - scale plasmid purification procedure. Bacteria picked from a freshly grown colony were used to inoculate 5ml of LB broth. The culture was incubated in the presence of the appropriate antibiotic for 12-16 hours. at 37°C with shaking at 220 cycles/minute, and used to inoculate 300ml LB broth (large-scale plasmid pREParation). The culture was incubated in the presence of the appropriate antibiotic for 12-16 hours at 37°C with shaking at 300 cycles/minute The large-scale extraction of plasmid DNA was performed using the Qiagen MaxipREP™ DNA Purification system, according to the manufacturer's instructions.

Plasmid DNA was then used to transform the Phoenix ecotropic retroviral packaging cell line. Phoenix ecotropic and amphotropic packaging cell lines were kind gifts from Dr Gary Nolan, Stanford University, California, USA (Grignani et al., 1998; Kinsella and Nolan, 1996). These cell lines are second generation retrovirus-producer lines for the production of helper-free ecotropic and amphotropic retroviruses. The lines are based on the 293T cell line, a human embryonic kidney cell line. They produce gag-pol genes with hygromycin as a co-selectable marker, and envelope proteins (either ecotropic or amphotropic) with diphtheria toxin as a second selectable marker. These cell lines were routinely maintained in DMEM supplemented with 10% FCS. They were additionally passaged for one week, about every 10 to 12 weeks, in 300 $\mu$g/ml hygromycin and 1 $\mu$g/ml diphtheria toxin for reselection.

Transfection of the Phoenix ecotropic retroviral packaging cell line with recombinant plasmid pREP8mb-SCF was performed by calcium phosphate precipitation. Briefly, 12$\mu$g plasmid DNA in 500$\mu$l 250mM CaCl$_2$ was added dropwise to an equal volume of 2x HBSS, while bubbling air through. The resulting solution was incubated at room temperature for 20-30 minutes, allowing a fine precipitate to form, then added dropwise to cells grown to 60% confluence in a 75 ml tissue culture flasks. The cells were maintained at 37°C, in 5% $CO_2$ in air for 24 hours before the calcium phosphate containing media was removed. The cells were washed twice with 10% culture medium, resuspended with fresh 10% culture medium and incubated for a further 24 hours at 37 °C, in 5% $CO_2$ in air. Selection of stable transformants was carried out in the presence of 2.5 mM histidinol. Resistant cells were propagated by splitting 1:5 the contents of 80% confluent flasks. Cells were kept in selection for at least 6-8 weeks. Stable transfectants were cryopreserved for further experiments.

The above stable cells obtained after 6-8 weeks were further subjected to dilution cloning. Briefly, 500 cells were seeded in 100 µl of DMEM supplemented with 10% FCS and 2.5 mM histidinol, in a 96-well flat-bottom plate. Four wells that reached confluence within a week were trypsinised and transferred into 48 well plates containing 300 µl DMEM supplemented with 10% FCS and 2.5 mM histidinol for a further week. These clones were then propagated into T25 flasks containing 10 ml of DMEM supplemented with 10% FCS and 2.5 mM histidinol. The four individual clones were then subjected to further experiments to determine the presence and expression of the mb-SCF in these cells.

*2. Detection of pREP8mb-SCF plasmid in the transformed Pheonix cell lines*

Retroviral packaging cells that were stably transfected with pREP8mb-SCF plasmid were confirmed to contain the plasmid by PCR. Briefly, clones growing in T25 flasks containing DMEM supplemented with 10% FCS and 2.5 mM histidinol, were trypsinised. The trypsinised cells were washed twice with PBS and resuspended in 500 µl of PBS. Two microlitres of the cell suspension was used as a template in a standard hot start PCR (see above) containing pREPF and EBV R primers.

| PREP F | GCTCGATACAATAAACGCC |
|--------|---------------------|
| EBV R  | GTGGTTTGTCCAAACTCATC |

PCR products were run on a 2% agarose gel containing 0.05% ethidium bromide. Positive controls for the amplification of the plasmid DNA were pREP8mb-SCF DNA itself as template DNA. Negative controls involved the use of water or untransfected cells as template in a standard PCR. The detection of pREP8mb-SCF in the packaging cells using primers pREPF and EBVR, gave rise to an amplification product of 1164 bp (Figure 3).

*3. Expression of mb-SCF proteins on engineered retroviral packaging cells.*

A standard indirect immunofluorescence assay was employed to detect the cell surface expression of SCF by the engineered packaging cells. The stably transfected packaging cells were grown on glass cover slips. When the cells were about 70% confluent, the cover slips were removed from the incubator and the cells fixed with methanol for 10 minutes at room temperature. The cells were then incubated with anti c-kit ligand/stem cell factor antibody (goat anti human SCF) (R&D Systems) at 1:5 dilution at 37 °C for 30 minutes. The cover slips were washed twice with PBS for 10 minutes and incubated with goat anti human anti IgG FITC conjugated secondary antibody. The cover slips were washed twice with PBS for 10 minutes and mounted onto a glass slide with 10%PBS/90% glycerol mountant and viewed under an Olympus UV microscope. Engineered packaging cells expressed high levels of SCF polypeptide as seen by intense fluorescent staining while non-engineered cells did not fluoresce (Figure 4). Antibody specificity was also confirmed by the use of non-immune goat serum on engineered packaging cells, which did not give a signal. No differences were seen in the signal intensities and number of fluorescing cells among the four separate clones.

The four modified packaging cell clones arising from histidinol selection were also analysed by flow cytometry to demonstrate the presence of mb-SCF. Briefly, transfected and untransfected cells growing in a T25 flask were detached using cell disassociation buffer, enzyme-free, PBS based (Gibco). Cells were washed twice in PBS. These cells were then incubated with a 1:5 dilution of anti c-kit ligand/stem cell factor antibody (goat anti human SCF) (R&D Systems) for 40 minutes at 4 °C. Cells were then washed twice in PBS and incubated with goat anti human anti IgG FITC conjugated secondary antibody for 30 minutes at 4°C. Cells were washed twice and fixed for 20 min at room temperature using 35 µl Leucoperm Reagent A (Serotec). Cells were resuspended in 1 ml of PBS for flow cytometry analysis. Flow cytometric analysis was carried out in the Stem Cell Lab, Hammersmith Hospital, using a Beckman FACScan machine. The software used to acquire and analyse the data was Cellquest. Forward and side scatter properties of untransfected cells were determined, and fluorescence intensities of transfected cells compared to untransfected cells stained with the both the primary and secondary antibodies. On this analysis, the four clones appeared to have slight differences in the frequency of cells expressing mb-SCF ranging from 73-98%. The expression levels of mb-SCF as determined by the peak on the fluorescence axis were the same for all four clones. The highest mb-SCF expressing clone, Clone 3, which had 98% expression of mb-SCF was selected for retroviral production and is shown in Figure 5. This clone was also confirmed to have high levels (97%) surface expression of CD59 as also shown in Figure 5.

**Example 3: Production of retroviruses from engineered retroviral packaging cells containing mb-SCF as part of the viral envelope**

A cDNA clone coding for the human mb - SCF polypeptide was isolated and cloned into a mammalian expression vector as described above. We then expressed the mb - SCF on the surface of a packaging cell line. As a next step we wished to determine whether viral particles derived from the retroviral packaging cell line contained the mb - SCF as part of the viral envelope.

*1. Production of retroviruses from the engineered Phoenix cells*

The Phoenix ecotropic cell line, the mb-SCF modified ecotropic Clone 3 and the amphotropic packaging cell lines were subsequently calcium phosphate transfected with the PINCO plasmid vector encoding the viral LTR, the packaging signal and a reporter gene, enhanced green fluorescence protein (eGFP) (Grignani *et al.,* 1998). The PINCO vector contains the full length Moloney murine leukaemia virus LTRs, the extended ψ sequence and a transcriptional cassette composed of the CMV promoter and the cDNA for the Enhanced Green Fluorescent Protein (eGFP) In addition to the retroviral backbone, this vector also contained the EBV oriP and EBNA-1 gene enabling the vector to replicate as an episome. This vector was also a kind gift from Dr Gary Nolan (Stanford University, Leyland, USA). Transient retrovirus supernatant was obtained prior to puromycin (1 μg/ml) selection. Following the removal of the calcium phosphate-DNA mixture and the overnight recovery of the packaging cells in complete medium, the medium was removed and replaced with 5 ml of fresh medium and the cells were incubated at 37 °C. Virus-laden supernatant was collected after 24 hours, 0.45 μ filter sterilised, aliquoted and stored at -70 °C. The PINCO transfected cells were propagated for a further 6 weeks in selection conditions with 1 μg/ml puromycin. Retrovirus was harvested in the same way as the transient supernatant.

*2. Calculation of viral titre from mb-SCF expressing cells*

Expression of the eGFP reporter gene was analysed by flow cytometry analysis of transduced NIH3T3 cells to obtain the retroviral titre. These cells were already available in the laboratory and were used to determine retroviral titres. They are maintained in DMEM supplemented with 10% FCS. Retroviral titres from transient and stable transfection of the PINCO vector into the three retroviral packaging cell lines (Phoenix Amphotropic, Ecotropic and Mb-SCF modified Ecotropic) were determined on NIH 3T3 cells. NIH 3T3 ($5 \times 10^4$ ) cells were seeded in a 24 well plate. One ml of an infection cocktail, containing retroviral supernatants ranging from 1μl to 100μl, DMEM supplemented with 10% heat inactivated FCS and 8 μg/ml polybrene, was added to individual wells. Infection was carried out overnight at 37 °C. The following day, the infection cocktail was removed and 1 ml of fresh medim was added. Incubation was carried out at 37 °C for a further 48 hours. The cells were then trypsinsed, washed twice in PBS, and fixed with Leucoperm. The cells were than flow cytometrically analysed to determine the expression of eGFP in the NIH 3T3 cells. Scatter properties of uninfected NIH 3T3 cells were determined and retrovirally transduced cells were compared for fluorescence intensities. The titre was calculated by titration of 1, 10 and 100 μl viral supernatant (each in duplicate) in a total volume of 1 ml of infection cocktail (containing complete medium and 8 μg/ml polybrene). The positive cell frequency measured as the percentage of fluorescent cells was plotted against the volume of supernatant used. The slope of the graph represented the retroviral titre. Retroviral viral titres are expressed as infectious particles (IP) /ml.

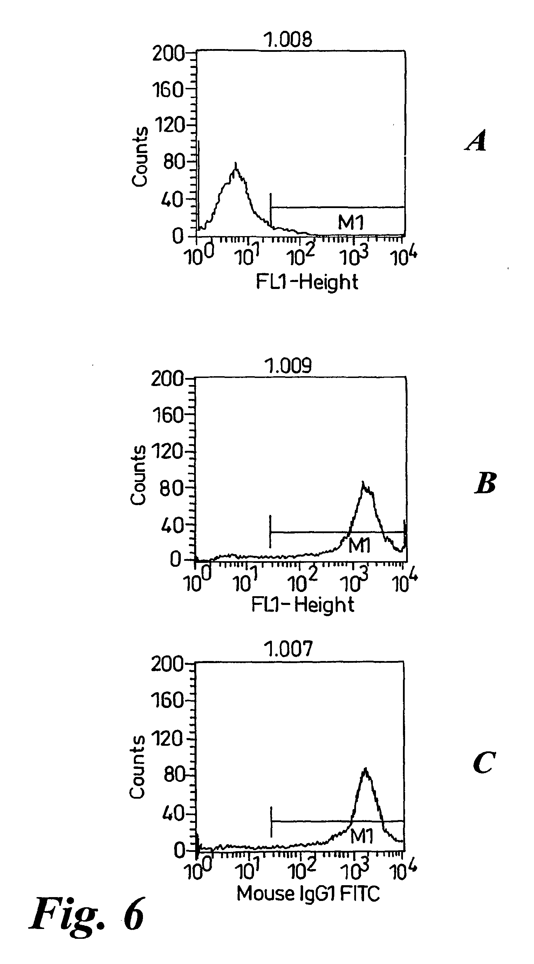

The retroviral titre of the mb-SCF engineered packaging cells are listed in Table 1. Retroviral titres between the unmodified ecotropic packaging cells and the mb-SCF modified packaging cells (stables) were not significantly different (Figure 6).

Table 1

| Packaging cell Line | Retroviral titre on NIH 3T3 cells (IP/ml ± sd) (n=3 experiments) |
|---|---|
| Phoenix Ecotropic | $3.88 \pm 1.18 \times 10^5$ |
| Phoenix mb-SCF modified (transient) | $2.98 \pm 0.32 \times 10^4$ |
| Phoenix mb-SCF modified (stable) | $2.60 \pm 0.53 \times 10^5$ |
| Phoenix Amphotropic | $2.08 \pm 1.29 \times 10^5$ |

*3. Detection of mb - SCF protein in retroviral particles derived from mb-SCF expressing cells*

Retroviruses were produced transiently and stably from the engineered ecotropic packaging cell, Clone 3, non-engineered ecotropic packaging cells and amphotropic packaging cells using the PINCO retroviral vector plasmid. Retroviral supernatant was collected from transiently transfected cells 48 hours post transfection with the PINCO plasmid and from stable transfectants that had been selected for 6 weeks in puromycin-containing medium. Four ml of these viral supernatants were pelleted by ultracentrifugation at 30,000 rpm for 1.5 h at 4 °C in a SW 55 Ti rotor (Beckman). The supernatant was discarded and the pelleted retroviral particles were resuspended in 50 μl of PBS and stored at -70 °C.

The pelleted retrovirus particle protein concentration was determined by an assay similar to the well documented Lowry assay, using a commercially available kit (Bio-Rad DC Protein Assay (Bio-Rad) ). Protein standards consisting of bovine serum albumin in the range of 0.2 mg/ml to 2 mg/ml were pREPared. Five microlitres of protein standards and samples (retroviral particles) were then assayed according to manufacterer's instructions using a Microplate Assay protocol. Protein concentrations of retroviral particles and protein standards were determined by absorbance at 750 nm.

Equal amounts of proteins were loaded gels for the detection of mb-SCF and CD59 in the particles by western blot analysis. 10μg of retroviral particles were mixed in 2x gel loading buffer (containing 50 mM Tris Cl, pH6.8, 2% SDS, 10 % glycerol and 1mg bromophenol blue), 0.1 M dithiothreitol and boiled for 5 minutes at 95 °C. This protein extract was then separated on a 12 % polyacrylamide gel under reducing conditions. The protein extracts were electrophoresed in parallel with prestained protein markers in 1x running buffer (25 mM Tris pH8.3), 250 mM glycine and 0.1% SDS) at 80 V for about 3-4 hours until the bromophenol marker was about 1 cm from the bottom of the gel. The proteins were transferred to a nitrocellulose membrane (Immobilon) using a semi-dry transblotting apparatus (Bio-Rad). One x transfer buffer was the same as the 1x running buffer but contained 20% methanol. The transfer of proteins was carried out according to manufacturer's instructions, running at a constant current of 0.8 mA/cm$^2$ for 1 hour. The success of transfer was determined by the detection of the prestained protein markers on the membrane, and the positions of these protein standards were marked with a pencil. The membrane was blocked with 10% milk protein (Marvel) in 1x TBST (Tris Buffered Saline and Tween20) for 1 hour at room temperature with gentle agitation. The demonstration of SCF on the retroviral particles was carried out by incubating, the membrane with goat anti-human SCF antibody (R&D Systems) at 1:500 dilution in 1x TBST containing 10% Marvel, at 4 °C for 18 hours. Human CD59 proteins on retroviral particles were demonstrated by incubating the membrane with mouse anti-human CD59 antibody (Serotec) at 1:250 dilution in 1x TBST containing 10% Marvel, at 4 °C for 24 hours. Antibody-treated membranes were then washed 3 times for 20 minutes in copious volumes of 1xTBST and a final wash for 20 minutes with 1xTBS. The SCF and CD59 antibodies were then detected by incubation with secondary donkey anti-goat IgG horse radish peroxidase (HRP) at a dilution of 1:2500 for 1 hour in 1x TBST containing 10% Marvel; and at a dilution of 1:500 secondary chicken anti-mouse IgG HRP, respectively. Membranes were subsequently washed 3 times for 20 minutes in copious volumes of 1x TBST and a final wash for 20 minutes with 1x TBS. SCF and CD59 antibodies were visualised using an enhanced chemiluminescence's kit (Amersham) according to manufacturer's instructions. Membranes reacted with the appropriate substrates from the kit, were then subjected to autoradiography. Autoradiography was conducted using different exposure times, ranging from 30 sec to 5 minutes, to obtain the best signals. Exposed autoradiography films were developed using an automatic film developer.

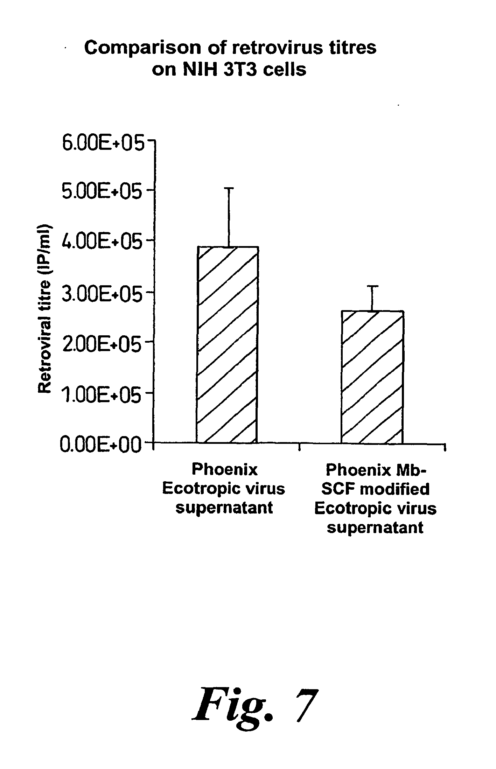

Retroviruses produced following transient transfection and stable producers were found to express the engineered mb-SCF, with the transient supernatant appearing to have a slightly higher level of expression (Figure 7). The non-engineered cells did not express the mb-SCF as expected. CD 59, a naturally occurring membrane protein in the packaging cells (engineered and non-engineered) could also be demonstrated.

**Example 4: Functionality of the mb - SCF containing viral particles.**

We have demonstrated above that it is possible to insert a membrane-bound polypeptide expressed in a viral packaging cell line into the surface envelopes of virus particles derived from that cell line. We now wished to determine whether the incorporated polypeptide retained biological activity. The mb - SCF polypeptide can bind to the c-kit receptor protein found at the cell surface of quiescent cells (Sehgal *et al.,* 1999). Once bound it can induce the cells to proliferate (Sehgal *et al.,* 1999). Hence we measured the capacity of the modified viral particles to bind to and induce proliferation of cell lines expressing the *c-kit* receptor.

*1. Binding of the mb - SCF viral particles to c-kit receptor positive cells*

To show that retroviral particles expressing mb-SCF could specifically bind to c-kit expressing cells, retrovirus binding experiments, as demonstrated by flow cytometry using the anti-SCF monoclonal antibody, were also carried out. The human megakaryoblastic leukaemia cell line, Mo7e, has been used in retroviral targeting experiments as the cell line has been reported to express high levels of c-kit (the receptor for SCF). Mo7e cells were maintained RPMI supplemented with 20% FCS and 10 ng/ml GM-CSF. Mo7e cells were incubated with 100 $\mu$l retroviral supernatant at 32° C for 1 hr. Cells were washed twice in cold PBS. One hundred microlitres of monoclonal antibody, 83A25 were then incubated with cells at 4 °C for 1 hour. These cells were washed twice with cold PBS. Cells were finally incubated with a FITC-conjugated rat anti-mouse IgG antibody for 1 hour at 4 °C. The cells were then washed twice in cold PBS and analysed by flow cytometry.

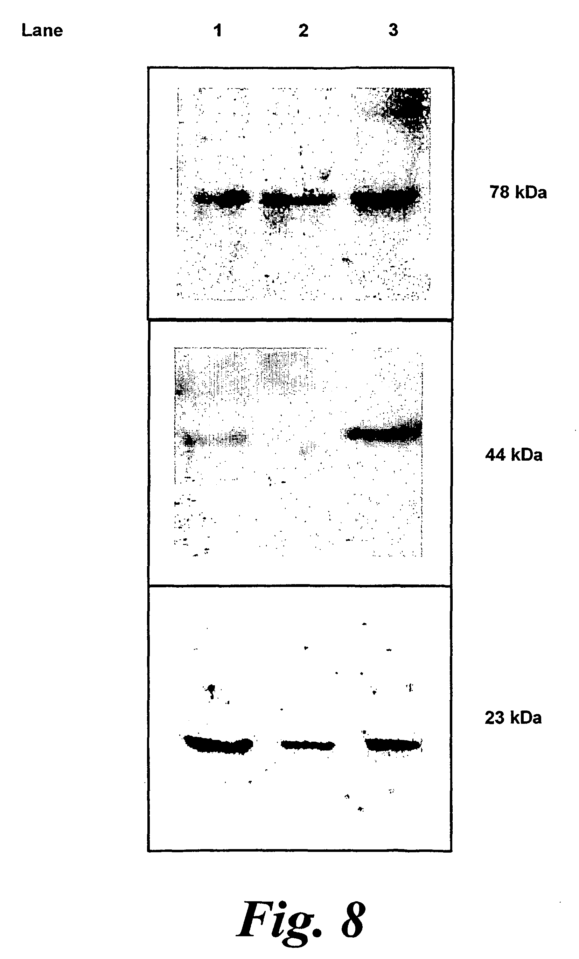

The mb-SCF retrovirus bound to the Mo7e cells whereas the unmodified ecotropic virus did not. Prior down regulation of the kit receptor by growing the Mo7e cells overnight in SCF instead of GM-CSF (confirmed by flow cytometry) abrogated binding of the mb-SCF retrovirus (Figure 8). These experiments demonstrate that targeted retroviral binding was achieved, resulting from the modification of the packaging cells.

*2. Mo7e cell proliferation following exposure to the modified viral particles.*

The biological activity of retroviral particles containing the mb-SCF proteins was demonstrated using a standard commercially available proliferation assay, CellTiter 96® AQ$_{ueous}$ One Solution Cell Proliferation Assay (Promega). Mo7e cells were washed twice in PBS and resuspended in RPMI supplemented with 20% FCS. 5000 cells per 90 $\mu$l or per 70 $\mu$l of resuspended media, were dispensed into 96 well round bottom microtitire plates. Ten $\mu$l or 30 $\mu$l of retroviral supernatant (prepared as above) from the Phoenix ecotropic mb-SCF cell lines, was added to the wells of the 96-well round bottom plates to bring the total volume of cell suspension to 100 $\mu$l. Positive controls for the proliferation assay included the use of 100 ng/ml recombinant purified human SCF. Negative controls included no cytokines or viral supernatants from the Phoenix ecotropic cells. Eight identical plates (each concentration in triplicate) were set up, with the first plate being assayed for background proliferation activity after two hours of having being set up.

Proliferation was measured every 24 hours by the addition of 20 $\mu$l of a solution containing, tetrazolium compound; 3-(4,5-dimethylthiazol-2-yl)-5-(3-carboxymethoxyphenyl)-2-(4-sulfophenyl)-2H-tetrazolium, inner salt (MTS) and an electron coupling reagent, phenazine ethosulfate (PES) to each well, and incubation for 1.5 hours, at 37 °C in a humidified incubator. The MTS compound is bioreduced by cells into a coloured formazan product that is soluble in tissue culture medium. The colour reaction was quantified by recording the absorbance at 490 nm with a 96-well plate reader.

Viral particles from mb-SCF engineered paakaging cells were able to stimulate the proliferation of Mo7e cells. Control retroviral particles from unmodified ecotropic packaging cells and cells incubated without the addition of cytokines did not stimualte the Mo7e cells. It is intresting to note that the proliferation from the mb-SCF modifed retrovirus begun only after day 3 of the experiment. Increasing the volume (to 30 $\mu$l) of retroviral supernatant did not appear to increase the rate of proliferation of the Mo7e cells. Retrovirus obtained transiently, was also capable of stimulating the proliferation of Mo7e cells. The positive control for the expriment was 100 ng/ml recombinant human SCF, which as expected stimulated the Mo7e cells. The results are plotted in Figure 9.

**Example 5: Gene transfer using viral particles of the invention and eGFP as a reporter gene.**

The examples above demonstrate that the methods of the invention can be used to incorporate a biologically active passenger peptide binding moiety into a viral particle. Here we show that such viral particles are capable of transducing target cells bound by the passenger peptide binding moiety.

**Methods**

**Retroviral Vector Plasmid, PINCO**

The retroviral vector used to generate retrovirus from the Phoenix retroviral packaging cells (amphotropic and ecotropic) was called PINCO (Grignani et al., 1998; Kinsella and Nolan, 1996). The PINCO vector contains the full length Moloney LTRs, the extended $\psi$ sequence and a transcriptional cassette composed of the CMV promoter and the cDNA for the Enhanced Green Fluorescent Protein (eGFP). In addition to the retroviral backbone, this vector also contained the EBV oriP and EBNA-1 gene enabling the vector to replicate as an episome. This vector was also a kind gift from Dr Gary Nolan (Stanford University, Leyland, USA).

## METHODS

### Primary cells

### Normal peripheral blood progenitor cells (PBPCs)

G-CSF mobilised peripheral blood from normal individuals obtained from the Stem Cell Laboratory, Hammersmith Hospital. These cells were acquired with signed informed consent and research ethics committee approval.

### Murine NIH 3T3 cell line

These cells were already available in the laboratory and were used to determined retroviral titres. They also were maintained in DMEM supplemented with 10% FCS.

### Human erythroleukaemia cell line TF-1 and Human megakaryoblastic leukaemia cell lines LAMA-84 and Mo7e

The TF-1 cell line, a human erythroleukaemia cell line was maintained in RPMI supplemented with 10% FCS and 1 ng/ml GM-CSF (Kitamura *et al.,* 1989). The human megakaryoblastic leukaemia cell line, Mo7e, was maintained RPMI supplemented with 20% FCS and 10 ng/ml GM-CSF. The human megakaryoblastic leukaemia cell line, LAMA-84, was maintained in RPMI supplemented with 10% FCS. These cell lines were used in retroviral targeting experiments as they have been reported to express high levels of c-kit (the receptor for SCF). The Mo7e cell line was also used in a proliferation assay to demonstrate biological activity of mb-SCF in retroviral particles. The Mo7e and LAMA-84 cell lines (Lee *et al.,* 2001; Miyazawa *et al.,* 1995; Seigneurin *et al.,* 1987) were kind gifts from Dr. Junia Melo. The TF-1 cell line was available in the laboratory.

### Supernatant transduction of haematopoietic cell lines

Multiplicity of infection (MOI) was calculated based on the retroviral titre on NIH3T3 cells and retroviral targeting experiments were carried out with an MOI of at least 2. An MOI of 1 represents one infectious retroviral particle per target cell. An infection cocktail containing retrovirus, 10 % FCS in DMEM and 8 $\mu$g/ml polybrene was incubated with 25,000 target cells for 24 hours at 37°C at 5% $CO_2$ in air. The medium was removed and replaced with fresh medium and incubated for a further 48 hours at 37 °C at 5% $CO_2$ in air. Target cells were then washed twice with PBS, fixed and assayed by flow cytometry.

### Co-culture of haematopoietic cell lines

Transduction of non-adherent haematopoietic cell lines by co-culture was performed with a 2-fold excess of producer cells. Retroviral producer cells were grown to confluence, trypsinised and irradiated at 2000 rads. Cells were replated into a 6-well microtitre plate, maintaining confluence, and incubated at 37°C in 5% $CO_2$ in air for 16-18 h. Cells were washed carefully in PBS and target cells (TF-1) in log-phase growth (5 x 10$^4$) were added in a total volume of 2 ml. A mock transduction in the absence of target cells was performed in parallel to control for residual producer cell activity. Cells were incubated at 37°C in 5% $CO_2$ in air for 24 hours. Non-adherent cells were removed, placed into a fresh 6-well microtitre plates and incubated for a further 8 h at 37°C, 5% $CO_2$ in air in order to facilitate adherence of contaminating producer cells prior to assay of vector reporter genes. Non-adherent cells were then resuspended in fresh medium and incubation carried out for a further 48 hours at 37°C, 5% $CO_2$ in air. Target cells were then washed twice with PBS, fixed and assayed by flow cytometric analysis.

### Retroviral transduction of primary cells

### Separation of mononuclear cells

Bone marrow cellsin preservative free heparin were diluted 1:4 in Hank's Buffered Salt Solution (Gibco). The diluted sample were layered over Lymphoprep (Nyegaard) and centrifuged at 1800 rpm for 30 minutes. Mononuclear cells (MNC) were removed from the interface and washed twice by centrifugation at 1800 rpm for 10 minutes in Hank's Buffered Salt Solution. MNC were counted using a haemocytometer chamber following dilution in 3% acetic acid to lyse any contaminating erythrocytes.

**CD 34 + cell separation**

CD34+ cells were separated from MNC using the MiniMacs™ system (Miltenyi Biotec) according to manufacterer's instructions. Briefly, MNCs were resuspended in a buffer containing of calcium-and magnesium-free PBS with 0.5% human serum albumin (Immune AG) and 5mM EDTA at 500 μl per $10^8$ MNC. One hundred μl each of reagants A1 (a blocking IgG) and A2 (QBEND/10- a mouse IgG recognising CD34) was added to the cells followed by incubation for 15 minutes at 4 °C. Cells were washed in 5 ml of buffer and resuspended in 400 μl of buffer per $10^8$ MNC. One hundred μl of reagent B (colloidal superparamagnetic MACS microbeads recognising reagent A2) was added to the cells and incubated for 15 minutes at 4 °C followed by another wash as above. The cells were resuspended in 500 μl of buffer then added to a prefilled MiniMacs™ column (type MS) held in a magnet and the cells allowed to pass through. Labelled CD34+ cells are retained in the column by the magnet. These cells are washed 4 times by adding 500 μl of buffer and allowing it to pass through. The column was removed from the magnet and the positive cells were eluted with 1 ml buffer. These cells were enumarated using a haemocytometer and the purity determined by staining a sample of cells with HPCA2 antibody (Beckton Dickinson), recognising a different epitope of the CD34 molecule, conjugated with FITC and FACS analysed. The purity of the samples was consistently above 90%.

**Retroviral transduction of CD34+ primary cells using the transwell technique**

The day before starting transduction of the CD34+ cells, the stable AM-12 retroviral producing cell line containing the amphotropic retroviral envelope and the retroviral genome (pBabeNeo; Morgenstern and Land, 1990), was plated in a 6-well plate at a concentration of 2 x $10^5$ cells/well and maintained in DMEM supplemented with 10% FCS. On the day of the transduction, the medium of the producer cell line was replaced with 2.5 ml of fresh DMEM supplemented with 10% FCS and protamine sulphate at a concentration of 8 μg/ml which would finally equilibrate to 4 μg/ml after the addition of the transwell. One 24 mm Transwell with a 0.4 μm membrane (Costar) was inserted into each well. CD 34+ ($10^5$) purified cells was resuspended in 2.5 ml of 30% FCS (Mycoplex™ PAA) and cytokine mix (50 ng/ml of IL-3, 100 ng/ml of SCF, 10 ng/ml of GM-CSF) in Iscoves Modified Dulbecco's Eagles Medium (Gibco) and transferred into the transwell inserts. The transduction process was carried out for 48 hours before the cells were harvested and cultured in semi-solid medium (methylcellulose), with and without G418 selection, for the growth of CFU-GM as described below.

**Clonogenic assay of retrovirally transduced progenitor cells**

**CFU-GM assay**

The CD34 + transduced cells were removed from the transwell and washed in Hank's Buffered Saline Solution. These cells were cultured in 3 ml methylcellulose (MC, Methocult H4230, Stem Cell Technologies) supplemented with 300 μl of CFU-GM mix (containing 50 ng/ml SCF, 1ng/ml GM-CSF, 10ng/ml IL-3 and 100 ng/ml G-CSF) and 1.5 mg/ml G418. After the cells were mixed well in MC, they were plated in 35 mm diameter petri dishes (Nunclon®) (1 ml /dish). The dishes in turn were placed in a 150 mm bacteriological petri dish (Sterilin) along with 35 mm petri dishes without lids containing sterile water to reduce the loss of culture medium through evaporation. The cultures were incubated at 37 °C in humidified 5 % $CO_2$ in air. CFU-GM colonies of more than 50 cells, were scored on day 7 of incubation using a Leica DM1L inverted microscope.

**Evaluation of gene transfer to primary cells**

**Transduction efficiency**

The efficiency of each CD34+ transduction experiment was calculated from the proportion of surviving CFU-GM colonies after 14 days of incubation in culture with G418 at a concentration of 1.5 mg/ml as described earlier.

$$\text{Transduction efficiency} = \frac{\text{No. of colonies surviving in G418}}{\text{No. of colonies without G418}} \times 100$$

**RESULTS AND DISCUSSION**

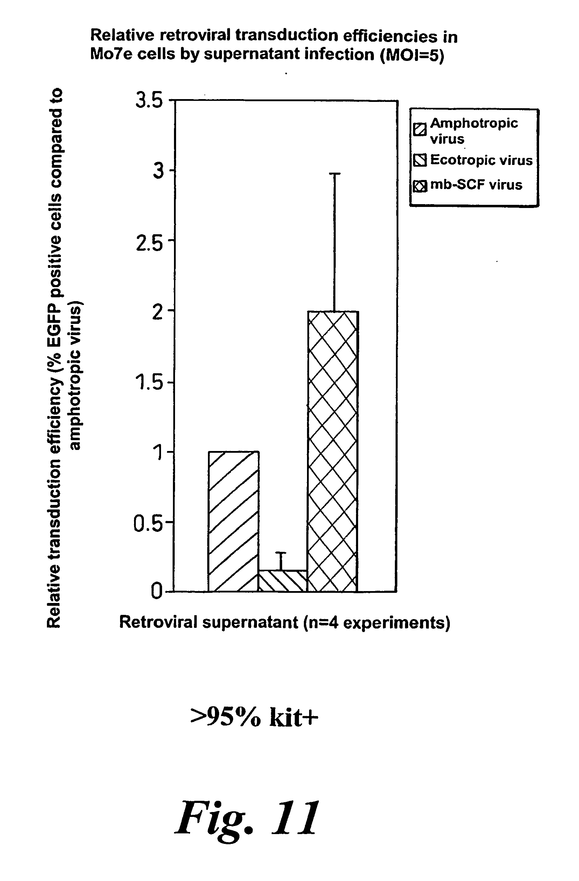

Experimental data is presented in Figures 11 to 17. All data have been expressed as efficiency of transduction relative to that obtained from the same viral vector packaged in an equivalent amphotropic packaging cell line i.e. Phoenix amphotropic packaging cells.

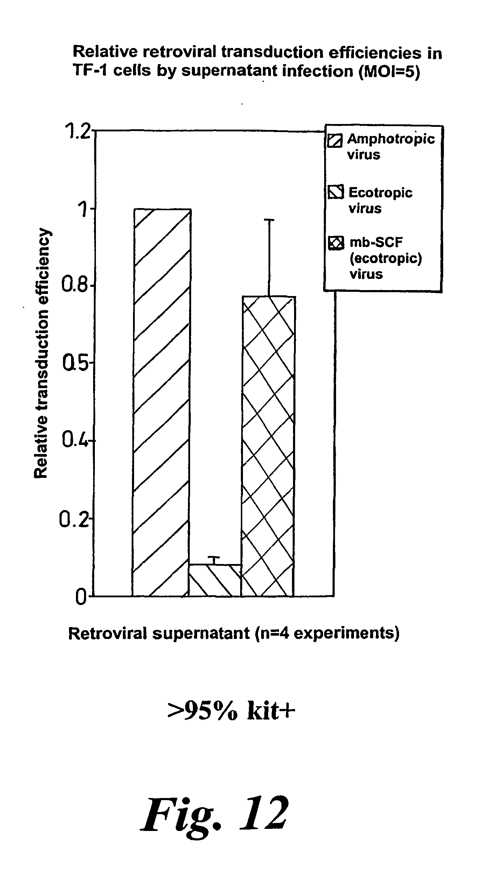

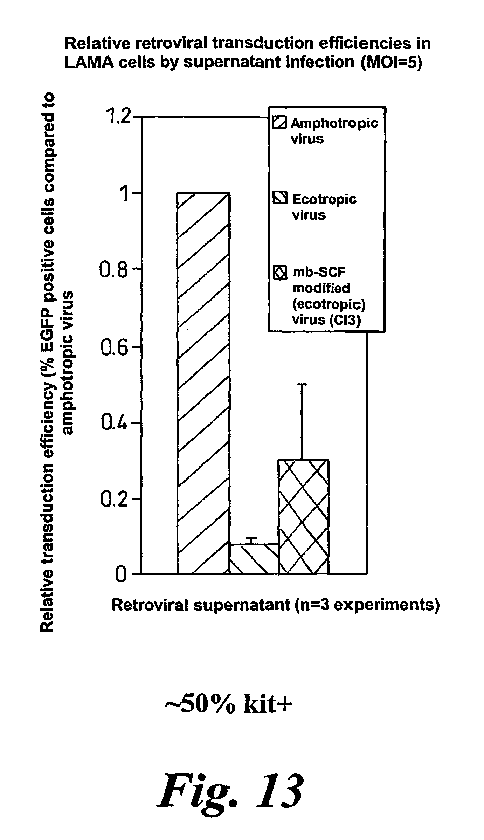

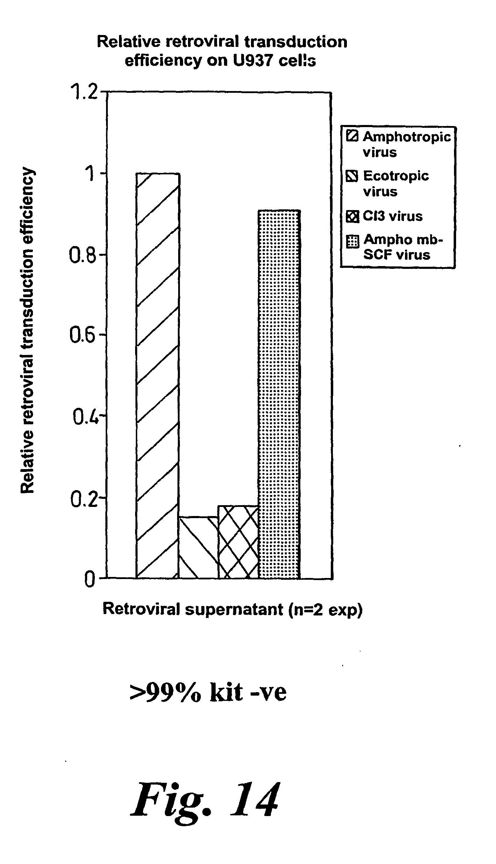

Figures 11 to 14 present data from viral particle transduction of three different SCF-receptor (kit) positive cell lines (Mo7e (Figure 11), TF-1 (Figure 12), LAMA (Figure 13)) and one kit negative (U937, Figure 14) cell line. Viral particles incorporating mb - SCF (labelled 'mb-SCF' in Figure 11 to 14) were prepared using the methods outlined in the above Examples. Ecotropic virus can only infect mouse cells, while amphotropic virus can infect both mouse and human cells.

As can be seen from Figures 11 to 14, viral particle transduction efficiency with unmodified ecotropic virus is very low for both kit positive and negative human cells. In contrast, transduction with ecotropic virus incorporating mb - SCF is highly efficient (similar efficiency to amphotropic), when target cells express high levels of kit (Mo7e and TF1 cells are >95% +ve for the presence of kit; Figures 11 and 12), somewhat less so when the kit expression is lower (LAMA cells are ~50% for the presence of kit +ve; Figure 13) and no different from unmodified ecotropic virus on kit negative cells (U937 cells; mb - SCF results labelled 'C13 virus'; Figure 14).

To demonstrate that the failure of the SCF-displaying viruses to transduce kit-negative cells is not a general defect of the ability of the viral particles to transduce these cells, data from experiments using amphotropic mbSCF - incorporating viral particles is shown as a control. Amphotropic mbSCF viral particles have a similar efficiency of transduction to unmodified amphotropic virus, and are capable of transducing kit negative cells (figure 14). Hence the modified viral particles incorporating the mb - SCF passenger binding motif are as capable of infecting susceptible cell types as normal viral particles.

Taken together, the data demonstrates that ecotropic viral particles incorporating the mb - SCF passenger peptide binding motif can transduce cells that have a receptor to which the mb - SCF peptide can bind. In contrast, ecotropic viral particles without the passenger peptide cannot transduce such cells. Hence, the method of the invention allows for viral particles to be produced that incorporate a passenger peptide binding motif that is capable of mediating target cell viral transduction. Such viral particles can be successfully used as a means for gene transfer to target cells.

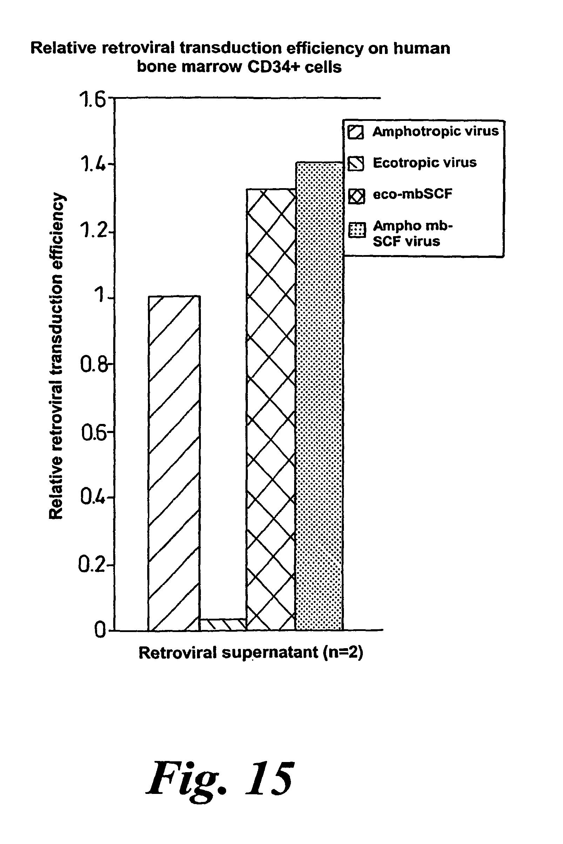

Figures 15 to 17 present data from the viral particle transduction of human bone marrow derived primary haematopoietic stem cells. Viral particles types are as those used in the experiments presented in Figure 11 to 14.

The transduction rate of human bone marrow derived primary haematopoietic stem cells (CD34+ cells; Figure 15) using viral particles incorporating the mb - SCF passenger peptide binding motif is equally, if not more, efficient than with amphotropic virus, whereas unmodified ecotropic virus, as expected, is very poor. Again, amphotropic mb - SCF - incorporating viral particles is highly efficient in this context, suggesting that the incorporation of mb - SCF on the virus can enhance transduction efficiency over conventional unmodified amphotropic virus.

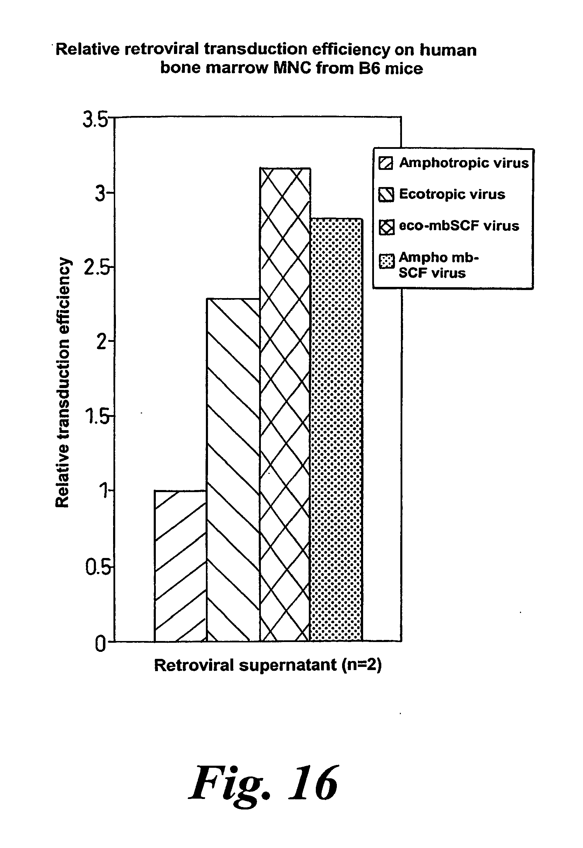

Figure 16 shows transduction of mouse bone marrow as a control. Here, we expect all viruses to transduce the target cells, and indeed, the ecotropic virus (specific for murine cells) is now superior to the amphotropic virus in efficiency. Both ecotropic mb - SCF and amphotropic mb - SCF viral particles, however, show enhanced levels of transduction, suggesting that the efficiency may be greater on kit positive cells, the receptor to which the mb - SCF passenger peptide binding motif binds. Human bone marrow derived primary haematopoietic stem cells will contain large numbers of cells with the kit receptor. It is worth noting that with the experiments with mouse cells there is no specific targeted transduction possible.

The experiments detailed above used the PINCO reteroviral vector which carries the enhanced green fluorescent protein (EGFP) gene and viral particle transduction of the cells was scored by analysis of fluorescence of cells using a FACs (fluorescence activated cell snorter).

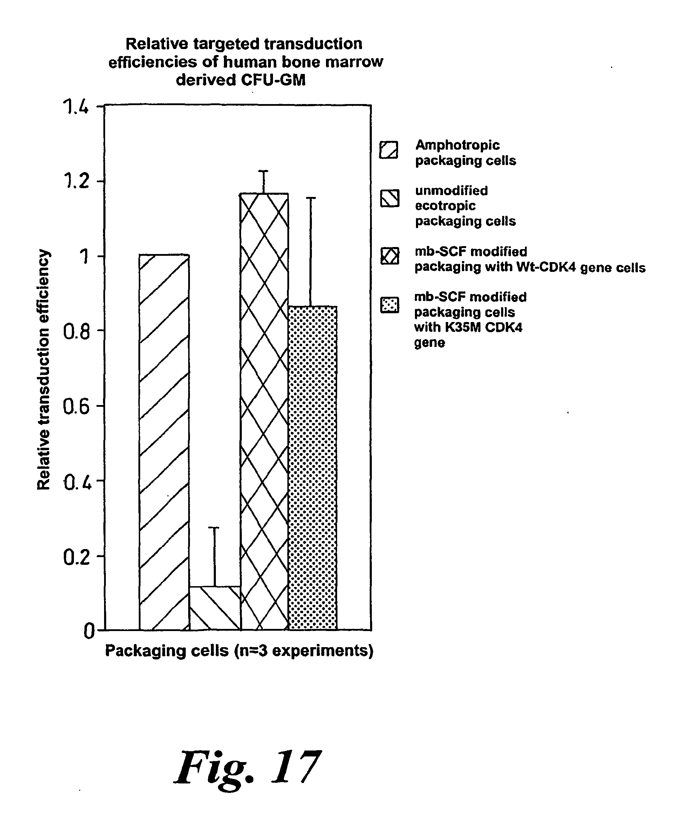

Finally, Figure 17 shows data from viral particle transduction of human bone marrow derived myeloid colony forming cells (CFU-GM). In these experiments the retroviral constructs carry a gene that confers resistance to the drug G418 (a derivative of the antibiotic neomycin, which can also kill mammalian cells). Bone marrow cell are transduced and then plated in semi-solid medium containing G418 to allow any resistant haematopoietic colonies to form. The colonies are scored after 14 days under an inverted microscope. Only successfully transduced cells should survive in the G418-medium to go on to produce colonies. The data are therefore derived from colony numbers obtained, following transduction with the different retroviruses and are again expressed relative to the performance of an equivalent amphotropic virus. The viral vector in these experiment was based on the pBabe vector (Morgenstern and Land, 1990).

As observed previously, unmodified ecotropic virus was very poor at transducing human haematopoietic progenitors - less than 10% of the rate or transduction mediated by amphotropic virus. In contrast, however, two different ecotropic mb - SCF constructs (labelled 'CDK4 gene' and 'KL35M CDK4 gene') gave highly efficient transduction of CFU-GM, a rate of transduction comparable to that observed using the amphotropic virus. Hence mb - SCF promotes ecotropic virus transduction of cells with the kit receptor.

These experiments confirm that viral particles incorporating the mb - SCF passenger peptide binding motif are capable of mediating target cell viral transduction. Such viral particles can be successfully used as a means for gene transfer to target cells.

**Example 6: Preparation of viral particles containing more than one engineered polypeptides in the viral envelope**

In Examples 1 and 2, a cDNA encoding a peptide to be incorporated into a viral particle was cloned from a human cell line and successfully expressed in a viral packaging cell line. Of course, it is possible to incorporate more than one peptide into a viral particle.

One a peptide to be used has been selected, for example, a peptide that is a ligand for a cell - type specific receptor, it is straightforward using the methods outlined in Example 1 to isolate a cDNA encoding the peptide from a population of cells in which the ligand is expressed. An alternative approach would be to conduct database searches to identify cDNAs encoding the ligand.

Once a cDNA encoding the ligand has been successfully cloned and sequenced to ensure it is the correct clone, the cDNA is cloned into a suitable mammalian cell expression vector. In Example 1, the mb - SCF cDNA was cloned into the pREP 8 mammalian expression plasmid from Invitrogen.. It has a histidinol resistance gene as a selectable marker for the expression of the desired cloned gene. Should a second peptide wished to be expressed in the same cell line, it is advisable to use an expression vector with an alternative resistance gene as a selectable marker to ensure both plasmids are present in the same cell line. Other suitable expression vectors include: pCMV - Script from Stratgene (G418 resistance), phCMV Xi - Clone from GTS (G418 resistance), pExchanger from Stratagene (hygromycin, puromycin or neomycin resistance) and pTriEx sysem from Novagen (hygromycin).

The expression vector with the cDNA is then transformed into the viral packaging cell line and transformants selected. Should the packaging cell line already have a peptide expression vector present, then transformants are selected for both the selectable marker genes. Example 2 demonstrates how a packaging cell line is transformed and successful transformants selected.

Example 3 outlines how retroviral particles are produced and collected from viral packaging cell lines containing the mb - SCF cDNA cloned into the pREP 8 mammalian expression vector. In this way, it would be possible to produce and collect viral particles having one or more peptides incorporated into the viral envelope.

**Example 7: Preparation of viral particles containing antibody fragments in the viral envelope**

It is possible to incorporate antibody fragments into the viral particles using, for an example, the procedure outlined below.

Single chain Fv from the mouse monoclonal antibody HMFG 1 and humanised monoclonal antibody Hu HMFG1

The nucleotide sequences encoding the $V_H$ heavy chains and $V_K$ light chains of HMFG1 and Hu HMFG1 are shown in Figure 15 of WO 94/10323 and are given in Verhoeyen et al (1993) Immunology 78, 364-370, both incorporated herein by reference.

Nucleotide and amino acid sequences of mouse and reshaped HMFG1 variable regions. (a) Heavy chain variable region sequences for mouse and reshaped HMFG1 (Mo $V_H$-HMFG1 and Hu $V_H$-HMFG1); (b) mouse and reshaped light chain variable regions respectively (Mo $V_K$-HMFG and Hu $V_K$-HMFG1). Amino acids numbering and definition of the CDR and framework regions are from Kabat et al (1987) Sequences of Proteins of Immunological Interest, Edn 4, US Dept of Health and Human Services Public Health Service, NIH, Bethesda, MD 20892, USA.

The methods described by Bird et al (1988) Science 242, 423 or Huston et al (1988) Proc. Natl. Acad. Sci. USA 85, 5879 are applied to the nucleotide sequences described in Figure 12 to generate genes encoding ScFv for HMFG1 and ScFv for Hu HMFG1. Using the recombinant DNA techniques described above it is straightforward to fuse a region of DNA encoding the ScFv for HMFG1 and ScFv for Hu HMFG1 to the membrane spanning region of mb - SCF in clone pRep8mb - SCF. Example 1 describes construction of pRep8mb - SCF. The membrane spanning region of SCF is from the proteolytic cleavage site coded by exon 6 to the C - terminus of the peptide (Hamel and Westphal, 1997).

The amino acid sequences of the $V_H$ and $V_L$ chains of H17E2 are disclosed in "Monoclonal antibodies - applications in clinical oncology", pages 37-43, 1991, A.A. Epenetos, ed., Chapman & Hall, UK.

Nucleotide sequences encoding the $V_H$ and $V_L$ chains are readily derived from the amino acid sequence using the genetic code and an ScFv can be made from the sequences using the methods of Bird *et al* or Huston *et al* as described above.

Once the mb - SCF - ScFv clones have been constructed they are transformed into a viral packaging cell line as outlined in Example 2. Viral particles incorporating the mb - SCF - ScFv peptide can be produced as outlined in Example 3.

Viral particle which have successfully incorporated the mb - SCF - ScFv peptide can be enriched from the total population of viral particles using immunological methods, well known to those skilled in the art. For example, an antibody that recognises the ScFv molecule can be conjugated to, for example, a column or a similar supporting matrix. Total viral particles are passed through the column, and only those viral particles that have incorporated the mb - SCF - ScFv peptide will be retained. After removal of the unbound viral particles, the bound viral particles can be released from the column. Alternative methods may be used wherein the column, or a similar supporting matrix, supports an antigen which the mb - SCF - ScFv peptide can bind to. One such antigen is the polymorphic epithelial mucin (PEM) core peptide encoded by *muc - 1*. Methods employing HMFG1 and Hu HMFG1 and/or using the epithelial mucin (PEM) core peptide are disclosed in WO 01/72336 and WO 94/10323, whose contents are herein incorporated by reference.

**Example 8: Use of viral particles of the invention for gene transfer.**

The ability of the viral particles of the invention to facilitate transduction of quiescent target cells is tested as described below.

The TF-1 cell line was developed from an erythroleukaemic patient and probably equates to cell arrested at an early stage of megakaryocyte development (Bagnis et al., 1994). The cells can only be grown in the presence of growth factors, the most usual being IL-3 or GM-OSF to which they respond very sensitively. They also are capable of dividing in response to SCF though this response is significantly weaker. These cells can easily be rendered quiescent by withdrawal of growth factor support. We used these cells as targets for retroviral transduction, following induction of quiescence.

In this example the viral packaging cell line can produce viral particles containing nucleic acid encoding the reported gene (β-galactosidase. This may be done by transfecting the same plasmid we had constructed containing the mbSCF cDNA (pRep8mb-SCF) into retroviral producer cells transducing a retroviral vector encoding the gene for β-galactosidase (NLSlacZ). This bacterial enzyme can be used in conjunction with synthetic substrates to produce a blue staining reaction when active enzyme is present. Accordingly, in these experiments, successfully transduced cells will stain blue following virally mediated gene transfer and is representative of showing transfer of genes encoding bioactive agents. The resulting producer line is identified as LacJP. Immunofluorescent staining of the LacJP cell line with anti-SCF antibody will show the presence of the surface bound SCF, as discussed in Example 2.

TF-1 cells in exponential growth are removed from growth factor and incubated overnight to allow them to become quiescent. TF-1 cells are co-cultured overnight with the retroviral particles incorporating mb-SCF as discussed above. Following co-culture, the TF-1 cells are removed, collected onto microscope slides and stained for β-galactosidase production.

Cells that are cocultured on the parent producer line will show no evidence for retroviral transduction. In contrast, some of the cells that are co-cultured with the SCF producer cells will be found to be positively stained. This can confirmed as cycling TF-1 cells by doubly-labelling the cells with titrated thymidine (3H). This radioactive nucleoside becomes incorporated into the DNA of dividing cells and can be detected by autoradiographic deposition of silver grains in a photographic emulsion into which the slides have been dipped. It is expected that many of the transduced TF-1 cells will also showed the presence of silver grains, indicating that cell division has taken place.

Thus, these experiments will show that expression of a surface bound growth factor by retroviral producer cell lines is able to facilitate the retroviral transduction of a quiescent target cell population and therefore enable the retroviral-mediated transfer of genes to cells that would normally be refractory to this technique.

**Example 9 Use of viral particles of the invention for gene transfer *in vivo***

The following example describes a procedure to use the viral particles of the invention for gene transfer. The viral particles are derived from a viral packaging cell which expresses a passenger peptide binding motif. The experiments listed below can be used to examine whether a viral particle incorporating, for example, a stem cell peptide binding motif can act as a gene transfer vector *in vivo*. Obviously, the tissues examined can be different depending on the cell type to which the viral particle is targeted.

Viral particles with β-*galactosidase* as a reporter gene for gene transfer, as described in Example 8, are injected in nude mice subcutaneously. Tissues are collected at 1 and 7 weeks after injection and expression of the β-galactosidase gene assessed by a histochemical assay described below.

**Histochemical staining for β-*galactosidase* activity in cells**

Cells transfected with combinations viral particles of the invention are washed with PBS and fixed in 0.1% glutaraldehyde in PBS for 10 minutes at room temperature, then washed twice in PBS, and incubated in X-gal solution (5mM $K_3Fe(CN)_6$, 5mM $K_4Fe(CN)_6:3H_2O$, 2mM $MgCl_2$, 200μg/ml 5-bromo-4-chloro-indolyl-β-D-galactopyranoside in N-N-dimethylfonmamide) for 20-24 hours at room temperature. Expression of the β-galactosidase gene product in cells can be detected microscopically by an indigo blue colour.

### Staining for β-galactosidase activity in whole tissues

Whole tissues from mice, immediately following dissection, are washed in PBS and fixed with a solution consisting of 1% formaldehyde, 0.2% glutaraldehyde, 2mM $MgCl_2$, 5mM EGTA, 0.02% NP-40. Excess fixative is removed after 1 hour by washing in PBS, and the sample incubated overnight in the dark with staining reagents (5mM $K_3Fe(CN)_6$, 5mM $K_4Fe(CN)_6$:$3H_2O$, 2mM $MgCl_2$, 0.02% NP-40, lmg/ml X-gal [5-bromo-4-chloro-3-β-D-galactopyranoside; Promega, Madison Wisc, USA]) in PBS. All procedures are carried out at room temperature. Samples can be examined by low power light microscopy and areas of blue stain scored in whole tissues, alternatively for confirmation, sections (1-2mm) can be excised from areas of stained tissue and observed at high magnification. The tissues that can be assayed for reporter gene expression include, for example, spleen, liver, kidney, brain, skin, heart, spinal chord, muscle and parotid gland.

The data will show the relative levels of blue coloration observed histochemically, and, hence, the degree of gene transfer.

Since sustained long-term expression is a desirable component of gene therapy, it is possible to attempt to assess this property in tissue culture, by monitoring β-*galactosidase* gene expression.

**Example 10 Gene targeting to cells of the central nervous system *in vivo* using viral particles of the invention.**

The following example describes a procedure to use the viral particles of the invention for gene transfer to cells of the central nervous system. The viral particles are derived from a viral packaging cell which expresses a passenger peptide binding motif that can specifically bind to cells of the central nervous system. Examples of possible peptides are well known to those skilled in the art, for example, antibodies against AMPA and NMDA receptors (Pickard *et al.,* 2000) or alpha(2A)-Adrenoreceptors (Hurt *et al.,* 2000).

Six week old female nude (nu/nu) mice are injected subcutaneously at the back of the neck with viral particles of the invention, as described above. Animals are killed by cervical dislocation 1 or 6 weeks post injection and major organs snap frozen and stored at -70°C. One quarter of each organ is washed in phosphate buffered saline (PBS) incubated in fix (1% formaldehyde, 0.2% gluteraldehyde, 2 mM $MgCl_2$, 5 mM EGTA, 0.02% Nonidet P40 in PBS), washed and incubated overnight at room temperature in X-gal solution (3 mM $K_4Fe(CN)_6$, 3 mM $K_3Fe(CN)_6$, 1 mM $MgCl_2$, 0.05% 5-bromo-4-chlor-3-indoly-β-D-galactopyranoside in PBS). Enzymatic activity, detected by deposition of blue product, is determined by eye or low power light microscopy.

The tissues that can be assayed for reporter gene expression include: spleen, liver, kidney, brain, skin, parotid gland. In particular, spinal tissues and other central nervous system tissues can be assayed

The data will show the relative levels of blue coloration observed histochemically, and, hence, the degree of gene transfer.

Using such a method the degree of specificity conferred by the peptide binding motif to the viral particle and the level of gene transduction can be assessed.

**Example 11. Other viral particles that can be used in the invention**

Whilst the foregoing examples used a retroviral vector based on the PINCO and pBabe MLV retrovirus, it will be appreciated that this was only for the purposes of illustrating the invention and was not meant to limit the invention. The following are examples of other viral vectors that can be employed in the method of the invention to produce viral particles with passenger peptide binding motifs.

LentiVector is a lentivirus - based vector system produced by Oxford BioMedica. They are based on HIV and Equine Infectious Anaemia Virus (EIAV)

pVPack is a MLV - based vector system produced by Stratagene.

The Pantropic Retroviral Expression System produced by BD Biosciences uses an envelope glyocporiten derived from vesicular stomatitis virus.

Retro - X system, again produced by BD Biosciences, is derived from the MLV virus.

Other examples will be within the knowledge of skilled persons.

**Example 12: Administration of viral particles of the invention to mammals.**

The aforementioned viral particles of the invention may be administered by any conventional method including oral and parenteral (eg subcutaneous or intramuscular) injection. A preferred method of administration is by means of a nasal inhalation spray, in which the viral particles are produced as in an aerosol. Such an approach has been used in the gene therapy of cystic fibrosis as an effective way of delivering the therapeutic agent to lung epithelial cells. The treatment may consist of a single dose or a plurality of doses over a period of time.

### *Pharmaceutical formulations*

Whilst it is possible for viral particles of the invention to be administered alone, it is preferable to present it as a pharmaceutical formulation, together with one or more acceptable carriers. The carrier(s) must be "acceptable" in the sense of being compatible with the viral particles of the invention and not deleterious to the recipients thereof. Typically, the carriers will be water or saline which will be sterile and pyrogen-free.

The following examples illustrate pharmaceutical formulations according to the invention in which the viral particles constitute the active ingredient'.

### Example A: Tablet

| | |
|---|---|
| Active ingredient | 100 mg |
| Lactose | 200 mg |
| Starch | 50 mg |
| Polyvinylpyrrolidone | 5 mg |
| Magnesium stearate | 4 mg |
| | 359 mg |

Tablets are prepared from the foregoing ingredients by wet granulation followed by compression.

### Example B: Ophthalmic Solution

| | |
|---|---|
| Active ingredient | 0.5 g |
| Sodium chloride, analytical grade | 0.9 g |
| Thiomersal | 0.001 g |
| Purified water to | 100 ml |
| pH adjusted to | 7.5 |

### Example C: Tablet Formulations

The following formulations A and B are prepared by wet granulation of the ingredients with a solution of povidone, followed by addition of magnesium stearate and compression.

### *Formulation A*

| | mg/tablet | mg/tablet |
|---|---|---|
| (a) Active ingredient | 250 | 250 |
| (b) Lactose B.P. | 210 | 26 |
| (c) Povidone B.P. | 15 | 9 |
| (d) Sodium Starch Glycolate | 20 | 12 |
| (e) Magnesium Stearate | 5 | 3 |
| | 500 | 300 |

*Formulation B*

|  | mg/tablet | mg/tablet |
|---|---|---|
| (a) Active ingredient | 250 | 250 |
| (b) Lactose | 150 | - |
| (c) Avicel PH 101® | 60 | 26 |
| (d) Povidone B.P. | 15 | 9 |
| (e) Sodium Starch Glycolate | 20 | 12 |
| (f) Magnesium Stearate | 5 | 3 |
|  | 500 | 300 |

*Formulation C*

|  | mg/tablet |
|---|---|
| Active ingredient | 100 |
| Lactose | 200 |
| Starch | 50 |
| Povidone | 5 |
| Magnesium stearate | 4 |
|  | 359 |

The following formulations, D and E, are prepared by direct compression of the admixed ingredients. The lactose used in formulation E is of the direction compression type.

*Formulation D*

|  | mg/capsule |
|---|---|
| Active Ingredient | 250 |
| Pregelatinised Starch NF15 | 150 |
|  | 400 |

*Formulation E*

|  | mg/capsule |
|---|---|
| Active Ingredient | 250 |
| Lactose | 150 |
| Avicel ® | 100 |
|  | 500 |

*Formulation F (Controlled Release Formulation)*

The formulation is prepared by wet granulation of the ingredients (below) with a solution of povidone followed by the addition of magnesium stearate and compression.

|  | mg/tablet |
|---|---|
| Active Ingredient | 500 |
| Hydroxypropylmethylcellulose (Methocel K4M Premium)® | 112 |
| Lactose B.P. | 53 |
| Povidone B.P.C. | 28 |
| Magnesium Stearate | 7 |
|  | 700 |

Drug release takes place over a period of about 6-8 hours and was complete after 12 hours.

Example D: Capsule Formulations

*Formulation A*

A capsule formulation is prepared by admixing the ingredients of Formulation D in Example C above and filling into a two-part hard gelatin capsule. Formulation B (*infra*) is prepared in a similar manner.

*Formulation B*

|  | mg/capsule |
|---|---|
| Active ingredient | 250 |
| Lactose B.P. | 143 |
| Sodium Starch Glycolate | 25 |
| Magnesium Stearate | 2 |
|  | 420 |

*Formulation C*

|  | mg/capsule |
|---|---|
| Active ingredient | 250 |
| Macrogol 4000 BP | 350 |
|  | 600 |

Capsules are prepared by melting the Macrogol 4000 BP, dispersing the active ingredient in the melt and filling the melt into a two-part hard gelatin capsule.

*Formulation D*

|                   | mg/capsule |
|-------------------|:----------:|
| Active ingredient | 250        |
| Lecithin          | 100        |
| Arachis Oil       | 100        |
|                   | 450        |

Capsules are prepared by dispersing the active ingredient in the lecithin and arachis oil and filling the dispersion into soft, elastic gelatin capsules.

*Formulation E (Controlled Release Capsule)*

The following controlled release capsule formulation is prepared by extruding ingredients a, b, and c using an extruder, followed by spheronisation of the extrudate and drying. The dried pellets are then coated with release-controlling membrane (d) and filled into a two-piece, hard gelatin capsule.

|                            | mg/capsule |
|----------------------------|:----------:|
| Active ingredient          | 250        |
| Microcrystalline Cellulose | 125        |
| Lactose BP                 | 125        |
| Ethyl Cellulose            | 13         |
|                            | 513        |

Example E: Injectable Formulation

| Active ingredient | 0.200 g |
|-------------------|---------|
| Sterile, pyrogen free phosphate buffer (pH 7.0) to 10 ml | |

The active ingredient is dissolved in most of the phosphate buffer (35-40°C), then made up to volume and filtered through a sterile micropore filter into a sterile 10 ml amber glass vial (type 1) and sealed with sterile closures and overseals.

Example F: Intramuscular injection

| Active ingredient      | 0.20 g  |
|------------------------|---------|
| Benzyl Alcohol         | 0.10 g  |
| Glucofurol 75®         | 1.45 g  |
| Water for Injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml glass vials (type 1).

Example G: Syrup Suspension

| Active ingredient | 0.2500 g |
|-------------------|----------|
| Sorbitol Solution | 1.5000 g |
| Glycerol          | 2.0000 g |

(continued)

| Dispersible Cellulose | 0.0750 g |
| Sodium Benzoate | 0.0050 g |
| Flavour, Peach 17.42.3169 | 0.0125 ml |
| Purified Water q.s. to | 5.0000 ml |

The sodium benzoate is dissolved in a portion of the purified water and the sorbitol solution added. The active ingredient is added and dispersed. In the glycerol is dispersed the thickener (dispersible cellulose). The two dispersions are mixed and made up to the required volume with the purified water. Further thickening is achieved as required by extra shearing of the suspension.

Example H: Suppository

| | mg/suppository |
|---|---|
| Active ingredient (63 $\mu$m)* | 250 |
| Hard Fat, BP (Witepsol H15 - Dynamit Nobel) | 1770 |
| | 2020 |

*The active ingredient is used as a powder wherein at least 90% of the particles are of 63 $\mu$m diameter or less.

One fifth of the Witepsol H15 is melted in a steam-jacketed pan at 45°C maximum. The active ingredient is sifted through a 200 $\mu$m sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250 $\mu$m stainless steel screen and, with continuous stirring, is allowed to cool to 40°C. At a temperature of 38°C to 40°C 2.02 g of the mixture is filled into suitable plastic moulds. The suppositories are allowed to cool to room temperature.

Example I: Pessaries

| | mg/pessary |
|---|---|
| Active ingredient | 250 |
| Anhydrate Dextrose | 380 |
| Potato Starch | 363 |
| Magnesium Stearate | 7 |
| | 1000 |

The above ingredients are mixed directly and pessaries prepared by direct compression of the resulting mixture.

Example J: Creams and ointments

(see Remington: The Science and Practise of Pharmacy, 19th ed., The Philadelphia College of Pharmacy and Science, ISBN 0-912734-04-3)

Example K: Microsphere formulations

The viral particles of the invention may also be delivered using microsphere formulations, such as those described in Cleland (1997), Pharm. Biotechnol. 10:1-43, Lee (2001) Curr. Opin. Biotechnol. 11:81-84, Cleland et al. (2001) J. Control. Release 72:13-24 and Takeuchi et al. (2001) Adv. Drug. Delic. Rev. 47:39-54.

Example L Dry Powder Inhalation

The viral particles of the invention may be delivered by inhalation, with the aid of a dry powder inhaler delivering micronised particles in metered quantities as described in Ansel (1999) Pharmaceutical Dosage Forms and Drug Delivery Systems, Lippincott Williams and Wilkins.

Example M Aerosol Inhalation

The viral particles of the invention may be delivered by inhalation, with the aid of a suitable inhaler delivering micronised particles in metered quantities employing a non CFC propellant as described in Ansel (1999) Pharmaceutical Dosage Forms and Drug Delivery Systems, Lippincott Williams and Wilkins.

The formulations may conveniently be presented in unit dosage form and may be prepared by any of the methods well known in the art of pharmacy. Such methods include the step of bringing into association the active ingredient (viral particles of the invention) with the carrier which constitutes one or more accessory ingredients. In general the formulations are prepared by uniformly and intimately bringing into association the active ingredient with liquid carries or finely divided solid carriers or both, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (eg povidone, gelatin, hydroxypropylmethyl cellulose), lubricant, inert diluent, preservative, disintegrate (eg sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide a desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations suitable for parenteral administration include aqueous and non-aqueous sterile injection solutions which may contain anti-oxidants, buffers, bacteriostatis and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use.

Extemporaneous injection solutions and suspensions may be prepared from sterile powders, granules and tablets of the kind previously described.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

**References.**

Ager et al., (1996) Hum Gene Ther 7, 2157 - 2164

Bagnis et al., (1994) Hum Gene Ther 5, 1325 - 1333

Beradi et al (1995) Science 267:104-108

Bodem et al (1997) Stem Cells 15 Supp 1, 141 - 147

Cadd et al., (1997) Bioessays 19, 993 - 1000

Cosman et al., (1988) Behring Inst Mitt 149 - 157

Cosset and Russell (1996) Gene Therapy 3, 946 - 956

Cosset et al., (1995) J Virol 69, 6314 - 6322

Cosset et al (1995) J Viro/ 69, 7430-7436.

Dimmock and Primrose, (1987) Introduction to Modem Virology Blackwell Scientific Publications, ISBN 0 - 632 - 01597 - 7

Etienne - Juan et al., (1992) J Gen Virol 73, 3251 - 3255

Garoff et al., (1998) Mirobiol Mol Biol Rev 62, 1171 - 1190

Goud et al., (1998) Virology 163, 251 - 254

Griffiths and Rottier, (1992) Semin Cell Biol 3, 367 - 381

Grignangi et al (1998) Cancer Res 58, 14 - 19

Hamel and Westphal, (1997) J Neuro - Oncology 35, 327 - 333

Han et al., (1995) Proc Natl Acad Sci USA 92, 9747 - 9751

Hatziioannou et al., (1998) J Virol 72, 5313 - 5317

Hatziioannou et al., (1999) Hum Gene Ther 10, 1533 - 1544

Hurt et al., (2000) J Biol Chem 275, 35424 - 35431

Kasahara et al., (1994) Science266, 1373 - 1376

Kinsella and Nolan (1996) Hum Gene Ther 7, 1405 -1413

Kitamura et al., (1989) Cell Physiol 140, 323 - 334

Lee et al., (2001) Biochem Biophys Res Commun 280, 675 - 683

Lee et al., (2002) Science, 297(5587):1689-92.

Lyman et al., (1995) Oncogene, 10, 149 - 157

Marin et al., (1996) J Virol 70, 2957 - 2962

Matano et al., (19950 J Gen Virol 76, 3165 - 3169

Miyazawa et al., (1995) Blood 85, 641 - 649

Morgenstern and Land, (1990) Nucleic Acid Res 18, 3587 - 3596

Neda et al., (1991) J Biol Chem 226, 14143 - 14149

Pickard et al., (2000) J Neurosci 20, 7922 - 7931

# EP 1 521 839 B1

Roux et al., (1989) Proc Natl Acad Sci USA 86, 9079 - 9083

Russell et al., (1993) Nucl Acid Res 21, 1081 - 1085

Russell et al., (1993) Nucleic Acid Res 21, 1081 - 1085

Sambrook et al., MOLECULAR CLONING: A LABORATORY MANUAL. 2001. 3rd edition Cold Spring Harbor Laboratory, Cold Spring Harbor, NY.

Schnierle et al., (1996) Gene Therapy 3, 334 - 342

Sehgal et al (1999) Gene Ther 6, 1084 -1091

Seigneurin et al., (1987) Exp Hematol 15, 822 - 832

Sellar et al., (2003) Nat Genet. 34(3):337-43

Somia et al., (1995) Proc Natl Acad Sci USA 92, 7570 - 7574

Strauss et al., (1995) Trends Microbiol 3, 346 - 350

Thalmeier et al., (1994) Blood 83, 1799 - 1807

Valsesia - Wittmann et al., (1994) J Virol 68, 4609 - 4619

Valsesia - Wittmann et al., (1996) J Virol 70 2059 - 2064

## Claims

1. A method of making an enveloped viral particle having a modified cell binding activity comprising:

   (i) providing a viral packaging cell containing viral nucleic acid encoding an enveloped viral particle having a first cell binding activity;
   (ii) the viral packaging cell also containing nucleic acid encoding a passenger peptide binding moiety wherein the passenger peptide binding moiety is provided at the outer plasma membrane of the cell and is a non-viral peptide other than a chimeric viral polypeptide made by fusing the native env portion of the viral peptide with a peptide binding moeity:
   (iii) the viral packaging cell line also comprising additional nucleic acid which can be expressed to provide a bioactive agent which is active in or on a target cell and wherein the bioactive agent either has a direct or indirect cytotoxic function; or is an enzyme capable of converting a relatively non-toxic pro-drug into a cytotoxic drug.
   (iv) expressing the viral nucleic acid and nucleic acid encoding the passenger peptide binding moiety so that a viral particle buds from a packaging cell membrane and the passenger peptide binding moiety is provided at a cell membrane such that the passenger peptide binding moiety is incorporated into the enveloped viral particle to modify its first cell binding activity;
   wherein the modified cell binding activity is that the viral particle can interact with one or more different cell types than that of the unmodified viral particle.

2. A method as claimed in Claim 1 wherein the enveloped viral particle is derived from a retroviral vector.

3. A method as claimed in any preceding claim wherein the passenger peptide binding moiety is a cell growth factor.

4. The method as claimed in Claim 3 wherein the growth factor is membrane-bound stem cell factor.

5. A method as claimed in Claims 1 or 2 wherein the passenger peptide binding moiety is an antibody, or an antigen binding fragment thereof.

6. A method as claimed in Claims 1 or 2 wherein the peptide binding moiety recognises a target cell-specific surface antigen.

7. A method as claimed in Claims 1 or 2 wherein the peptide binding moiety is at least part of a member of a binding pair comprising a target-cell specific cell-surface receptor and its ligand.

8. A method as claimed in any previous claim wherein the bioactive agent is of use in the prevention and/or treatment and/or diagnosis of a disease or disorder.

9. A method as claimed in any previous claim wherein the bioactive agent is any one of ricin; tumour necrosis factor; interleukin-2; interferon-gamma; ribonuclease; deoxyribonuclease; Pseudomonas exotoxin A; and caspase.

10. A method as claimed in any previous claim wherein the bioactive agent is either cytosine deaminase or thymidine kinase.

11. A method as claimed in any preceding claim wherein the modified cell binding activity allows the enveloped viral particle to bind to a target cell.

12. A method as claimed in Claim 11 wherein the target cell is a mammalian cell.

13. A method as claimed in Claim 11 wherein the target cell is a human cell.

14. A method as claimed in Claim 11 wherein target cell is a quiescent cell.

15. A method as claimed in Claim 11 wherein target cell is a human haematopoietic stem cell.

16. A method as claimed in Claim 11 wherein the target cell is a cancer cell.

17. A method as claimed in Claim 11 wherein the target cell is a mammalian T-cell.

18. An enveloped viral particle having a modified cell binding activity obtainable by a method as claimed in any preceding claim, the modified cell binding activity being conferred by a non-viral peptide other than a chimeric viral polypeptide made by fusing the native env portion of the viral peptide with a peptide binding moiety, and wherein the modified cell binding activity is that the viral particle can interact with one or more different cell types than those of the unmodified viral particle.

19. A enveloped viral particle having a modified cell binding activity obtained by a method as claimed in any of claims 1 to 17, wherein the modified cell binding activity is that the viral particle can interact with one or more different cell types than those of the unmodified viral particle.

20. A method of preparing an enriched population of a target cell type from a larger population of cells wherein: (1) enveloped viral particles of either of claims 18 and 19, having a modified binding activity for target cells, are exposed to a population of cells comprising the target cell type to permit binding to the viral particles; (2) enveloped viral particles bound to target cells are then separated from the population of cells; (3) optionally, the enveloped viral particles are subsequently removed from the target cells.

21. A method of enriching the titre of enveloped viral particles incorporating a passenger peptide binding moiety from a population of enveloped viral particles as claimed in claim 18 or 19 comprising:

   i) providing a support to which the passenger peptide binding moiety binds; and,
   ii) exposing the population of enveloped viral particles to the support; and, optionally,
   iii) isolating the enveloped viral particles which bind to the support from the viral particles which do not bind to the support.

22. A preparation of enveloped viral particles as claimed in claim 18 or 19 enriched for enveloped viral particles incorporating a passenger peptide binding moiety, the preparation having a titre of the enveloped viral particles of at least $10^5$ ifu/ml (infection forming units/ml).

23. The preparation as claimed in claim 22 further comprising a pharmaceutically acceptable excipient and/or carrier.

24. An enveloped viral particle as claimed in claim 18 or 19, or a preparation of enveloped viral particles as claimed in Claim 22 or 23 for use in medicine.

25. Use of an enveloped viral particle according to claim 18 or 19, or a preparation of enveloped viral particles as claimed in Claim 22 or 23, in the manufacture of a medicament for the diagnosis and/or prevention and/or treatment of a disease or a disorder.

26. Use of an enveloped viral particle according to claim 18 or 19, or a preparation of enveloped viral particles as claimed in Claim 22 or 23, in the manufacture of a medicament for the prevention and/or treatment of arthritis.

27. The use of claim 26 wherein the enveloped virus particle incorporates a binding molecule which binds to CD5 as a passenger peptide binding moiety.

28. The use of claim 26 wherein the enveloped viral particle incorporates membrane-bound stem cell factor as a passenger peptide binding moiety.

29. Use of an enveloped viral particle according to claim 18 or 19, or a preparation of enveloped viral particles as claimed in Claim 22 or 23, in the manufacture of a medicament for the diagnosis, and/or prevention and/or treatment of cancer.

30. The use of claim 29 wherein the cancer is ovarian cancer.

31. The use of claim 29 or 30 wherein the enveloped viral particle incorporates membrane-bound stem cell factor as a passenger peptide binding moiety.

32. The use of any of claims 29 to 31 wherein the enveloped viral particle includes a gene encoding a OPCML (opoid binding protein/cell adhesion molecule-like) polypeptide.

33. An enveloped viral particle according to claim 18 or 19, or a preparation of enveloped viral particles as claimed in Claim 22 or 23, for use in gene transfer.

34. Use of the enveloped viral particles according to claim 18 or 19, or a preparation of enveloped viral particles as claimed in Claim 22 or 23, in the manufacture of a vaccine.

35. A pharmaceutical composition comprising an enveloped viral particle according to any one of claims 18 or 19, or a preparation of an enveloped viral particles as claimed in Claim 22 or 23, and a pharmaceutically acceptable carrier.

**Patentansprüche**

1. Verfahren zur Erzeugung eines behüllten Viruspartikels mit einer modifizierten zellbindenden Aktivität, wobei das Verfahren umfasst:

i) Bereitstellen einer viralen Verpackungszelle, die virale Nucleinsäure enthält, die für ein behülltes Viruspartikel mit einer ersten zellbindenden Aktivität codiert,

ii) wobei die virale Verpackungszelle auch Nucleinsäure enthält, die für eine ein Passagierpeptid bindende Gruppe codiert, wobei die das Passagierpeptid bindende Gruppe an der äußeren Plasmamembran der Zelle bereitgestellt ist und ein nicht-virales Peptid ist, das kein schimärisches virales Polypeptid ist, das durch das Fusionieren des nativen env-Teils des viralen Peptids mit einer peptidbindenden Gruppe hergestellt ist,

iii) wobei die virale Verpackungszelllinie auch weitere Nucleinsäure umfasst, die zur Bereitstellung eines bioaktiven Agens exprimiert werden kann, das in oder auf einer Zielzelle aktiv ist, und wobei das bioaktive Agens entweder eine direkt oder eine indirekt zytotoxische Funktion hat oder ein Enzym ist, das in der Lage ist, ein relativ untoxisches Prodrug in ein zytotoxisches Arzneimittel zu überführen,

iv) Exprimieren der viralen Nucleinsäure und der Nucleinsäure, welche für die das Passagierpeptid bindende Gruppe codiert, sodass ein Viruspartikel aus einer Membran der Verpackungszelle knospt und die das Passagierpeptid bindende Gruppe an einer Zellmembran bereitgestellt wird, sodass die das Passagierpeptid bindende Gruppe in das behüllte Viruspartikel inkorporiert wird, wodurch seine erste zellbindende Aktivität modifiziert wird, wobei die modifizierte zellbindende Aktivität darin besteht, dass das Viruspartikel mit einem anderen Zelltyp oder mit mehreren anderen Zelltypen interagieren kann als das unmodifizierte Viruspartikel.

**2.** Verfahren, wie es im Anspruch 1 beansprucht wird, wobei das behüllte Viruspartikel von einem retroviralen Vektor stammt.

**3.** Verfahren, wie es in irgendeinem vorhergehenden Anspruch beansprucht wird, wobei die das Passagierpeptid bindende Gruppe ein Zellwachstumsfaktor ist.

**4.** Verfahren, wie es im Anspruch 3 beansprucht wird, wobei der Wachstumsfaktor der Membrane-bound Stem Cell Factor ist.

**5.** Verfahren, wie es in den Ansprüchen 1 oder 2 beansprucht wird, wobei die das Passagierpeptid bindende Gruppe ein Antikörper oder ein antigenbindendes Fragment von ihm ist.

**6.** Verfahren, wie es in den Ansprüchen 1 oder 2 beansprucht wird, wobei die das Peptid bindende Gruppe ein zielzellspezifisches Oberflächenantigen erkennt.

**7.** Verfahren, wie es in den Ansprüchen 1 oder 2 beansprucht wird, wobei die das Peptid bindende Gruppe wenigstens ein Teil eines Partners in einem Bindungspaar ist, das einen zielzellspezifischen Zelloberflächenrezeptor und seinen Liganden umfasst.

**8.** Verfahren, wie es in irgendeinem vorhergehenden Anspruch beansprucht wird, wobei das bioaktive Agens für die Prävention und/oder Behandlung und/oder Diagnose einer Krankheit oder Störung von Nutzen ist.

**9.** Verfahren, wie es in irgendeinem vorhergehenden Anspruch beansprucht wird, wobei das bioaktive Agens irgendeines von Ricin, Tumornekrosefaktor, Interleukin-2, Gamma-Interferon, Ribonuclease, Desoxyribonuclease, Pseudomonas-Exotoxin-A und Caspase ist.

**10.** Verfahren, wie es in irgendeinem vorhergehenden Anspruch beansprucht wird, wobei das bioaktive Agens entweder die Cytosindesaminase oder die Thymidinkinase ist.

**11.** Verfahren, wie es in irgendeinem vorhergehenden Anspruch beansprucht wird, wobei die modifizierte zellbindende Aktivität es dem behüllten Viruspartikel ermöglicht, an eine Zielzelle zu binden.

**12.** Verfahren, wie es im Anspruch 11 beansprucht wird, wobei die Zielzelle eine Säugerzelle ist.

**13.** Verfahren, wie es im Anspruch 11 beansprucht wird, wobei die Zielzelle eine humane Zelle ist.

**14.** Verfahren, wie es im Anspruch 11 beansprucht wird, wobei die Zielzelle eine ruhende Zelle ist.

**15.** Verfahren, wie es im Anspruch 11 beansprucht wird, wobei die Zielzelle eine humane hämatopoetische Stammzelle ist.

**16.** Verfahren, wie es im Anspruch 11 beansprucht wird, wobei die Zielzelle eine Krebszelle ist.

**17.** Verfahren, wie es im Anspruch 11 beansprucht wird, wobei die Zielzelle eine T-Zelle eines Säugetiers ist.

**18.** Behülltes Viruspartikel mit einer modifizierten zellbindenden Aktivität, das mittels eines Verfahrens erhalten werden kann, wie es in irgendeinem vorhergehenden Anspruch beansprucht wird, wobei die modifizierte zellbindende Aktivität durch ein nicht-virales Peptid bewirkt wird, das kein schimärisches virales Polypeptid ist, das durch das Fusionieren des nativen env-Teils des viralen Peptids mit einer peptidbindenden Gruppe hergestellt ist, und wobei die modifizierte zellbindende Aktivität darin besteht, dass das Viruspartikel mit einem anderen Zelltyp oder mit mehreren anderen Zelltypen interagieren kann als das unmodifizierte Viruspartikel.

**19.** Behülltes Viruspartikel mit einer modifizierten zellbindenden Aktivität, das mittels eines Verfahrens erhalten werden kann, wie es in irgendeinem der Ansprüche 1 bis 17 beansprucht wird, wobei die modifizierte zellbindende Aktivität darin besteht, dass das Viruspartikel mit einem anderen Zelltyp oder mit mehreren anderen Zelltypen interagieren kann als das unmodifizierte Viruspartikel.

**20.** Verfahren zur Präparation einer angereicherten Population eines Zielzellentyps aus einer größeren Population von Zellen, wobei 1) behüllte Viruspartikel nach einem der beiden Ansprüche 18 und 19 mit einer modifizierten bindenden Aktivität für Zielzellen gegen eine Population von Zellen exponiert werden, die den Zielzellentyp umfasst, um die Bindung an das Viruspartikel zu ermöglichen, 2) die an Zielzellen gebundenen behüllten Viruspartikel dann von der Population der Zellen abgetrennt werden, 3) gegebenenfalls die behüllten Viruspartikel anschließend von den Zielzellen entfernt werden.

21. Verfahren zur Erhöhung des Titers der behüllten Viruspartikel, die eine ein Passagierpeptid bindende Gruppe enthalten, aus einer Population behüllter Viruspartikel, wie sie im Anspruch 18 oder 19 beansprucht werden, das umfasst:

   i) Bereitstellen eines Trägers, an den die das Passagierpeptid bindende Gruppe bindet, und
   ii) Exponieren der Population der behüllten Viruspartikel gegen den Träger und, gegebenenfalls,
   iii) Isolieren der behüllten Viruspartikel, die an den Träger binden, aus den Viruspartikein, die nicht an den Träger binden.

22. Präparation behüllter Viruspartikel, wie sie im Anspruch 18 oder 19 beansprucht werden, angereichert an behüllten Viruspartikeln, die eine ein Passagierpeptid bindende Gruppe enthalten, wobei die Präparation einen Titer der behüllten Viruspartikel von wenigstens $10^5$ IFU/ml (Infection Forming Units/ml) aufweist.

23. Präparation, wie sie im Anspruch 22 beansprucht wird, die ferner einen pharmazeutisch annehmbaren Hilfsstoff und/oder Träger umfasst.

24. Behülltes Viruspartikel, wie es im Anspruch 18 oder 19 beansprucht wird, oder Präparation behüllter Viruspartikel, wie sie im Anspruch 22 oder 23 beansprucht wird, für die Verwendung in der Medizin.

25. Verwendung eines behüllten Viruspartikels gemäß Anspruch 18 oder 19 oder einer Präparation behüllter Viruspartikel, wie sie im Anspruch 22 oder 23 beansprucht wird, bei der Herstellung eines Medikaments für die Diagnose und/oder Prävention und/oder Behandlung einer Krankheit oder Störung.

26. Verwendung eines behüllten Viruspartikels gemäß Anspruch 18 oder 19 oder einer Präparation behüllter Viruspartikel, wie sie im Anspruch 22 oder 23 beansprucht wird, bei der Herstellung eines Medikaments für die Prävention und/oder Behandlung von Arthritis.

27. Verwendung nach Anspruch 26, wobei das behüllte Viruspartikel ein Bindungsmolekül enthält, das an CD5 als Passagierpeptid-bindende Gruppe bindet.

28. Verwendung nach Anspruch 26, wobei das behüllte Viruspartikel den Membrane-bound Stem Cell Factor als Passagierpeptid-bindende Gruppe enthält.

29. Verwendung eines behüllten Viruspartikels gemäß Anspruch 18 oder 19 oder einer Präparation behüllter Viruspartikel, wie sie im Anspruch 22 oder 23 beansprucht wird, bei der Herstellung eines Medikaments für die Diagnose und/oder Prävention und/oder Behandlung von Krebs.

30. Verwendung nach Anspruch 29, wobei der Krebs Ovarialkrebs ist.

31. Verwendung nach Anspruch 29 oder 30, wobei das behüllte Viruspartikel den Membrane-bound Stem Cell Factor als Passagierpeptid-bindende Gruppe enthält.

32. Verwendung von irgendeinem der Ansprüche 29 bis 31, wobei das behüllte Viruspartikel ein Gen enthält, das für ein OPCML (Opioid binding protein/cell adhesion molecule-like)-Polypeptid codiert.

33. Behülltes Viruspartikel gemäß Anspruch 18 oder 19 oder Präparation behüllter Viruspartikel, wie sie im Anspruch 22 oder 23 beansprucht wird, für die Verwendung beim Gentransfer.

34. Verwendung des behüllten Viruspartikels gemäß Anspruch 18 oder 19 oder einer Präparation behüllter Viruspartikel, wie sie im Anspruch 22 oder 23 beansprucht wird, bei der Herstellung eines Impfstoffs.

35. Pharmazeutische Zusammensetzung, die ein behülltes Viruspartikel gemäß irgendeinem der Ansprüche 18 oder 19 oder eine Präparation behüllter Viruspartikel, wie sie im Anspruch 22 oder 23 beansprucht wird, und einen pharmazeutisch annehmbaren Träger umfasst.

**Revendications**

1. Procédé de préparation d'une particule virale enveloppée présentant une activité de liaison cellulaire modifiée, comprenant :

   (i) l'obtention d'une cellule d'encapsidation virale contenant un acide nucléique viral codant une particule virale enveloppée présentant une première activité de liaison cellulaire ;
   (ii) la cellule d'encapsidation virale contenant également un acide nucléique codant un fragment peptidique de liaison passager, dans lequel le fragment peptidique de liaison passager se trouve sur la membrane plasmatique externe de la cellule et est un peptide non viral différent d'un polypeptide viral chimère formé par la fusion de la partie env native du peptide viral avec un fragment peptidique de liaison ;
   (iii) la lignée de cellule virale d'encapsidation comprenant en outre un acide nucléique supplémentaire pouvant être exprimé pour produire un agent bioactif qui est actif dans ou sur une cellule cible, et dans lequel l'agent bioactif soit présente une fonction cytotoxique directe ou indirecte ; soit est une enzyme capable de convertir une prodrogue relativement non toxique en drogue cytotoxique ;
   (iv) l'expression de l'acide nucléique viral et de l'acide nucléique codant le fragment peptidique de liaison passager pour qu'une particule virale pointe hors d'une membrane de cellule d'encapsidation et que le fragment peptidique de liaison passager se trouve sur une membrane cellulaire, de manière à ce que le fragment peptidique de liaison passager soit incorporé dans la particule virale enveloppée pour modifier sa première activité de liaison cellulaire ;
   dans lequel l'activité de liaison cellulaire modifiée rend possible l'interaction de la particule virale avec un ou plusieurs types cellulaires différents de celui de la particule virale non modifiée.

2. Procédé selon la revendication 1, dans lequel la particule virale enveloppée dérive d'un vecteur rétroviral.

3. Procédé selon l'une quelconque des revendications précédentes, dans lequel le fragment peptidique de liaison passager est un facteur de croissance cellulaire.

4. Procédé selon la revendication 3, dans lequel le facteur de croissance est un facteur de cellule-souche lié à la membrane.

5. Procédé selon la revendication 1 ou 2, dans lequel le fragment peptidique de liaison passager est un anticorps ou l'un de ses fragments se liant à un antigène.

6. Procédé selon la revendication 1 ou 2, dans lequel le fragment peptidique de liaison reconnaît un antigène de surface spécifique d'une cellule cible.

7. Procédé selon la revendication 1 ou 2, dans lequel le fragment peptidique de liaison est au moins une partie d'un membre d'une paire de liaison comprenant un récepteur de surface cellulaire spécifique d'une cellule cible et son ligand.

8. Procédé selon l'une quelconque des revendications précédentes, dans lequel l'agent bioactif est utilisé dans la prévention et/ou le traitement et/ou le diagnostic d'une maladie ou d'un trouble.

9. Procédé selon l'une quelconque des revendications précédentes, dans lequel l'agent bioactif est l'un quelconque choisi parmi la ricine ; le facteur onconécrosant ; l'interleukine 2 ; l'interféron gamma ; la ribonucléase ; la désoxyribonucléase ; l'exotoxine A de Pseudomonas ; et la caspase.

10. Procédé selon l'une quelconque des revendications précédentes, dans lequel l'agent bioactif est soit la cytosine désaminase soit la thymidine kinase.

11. Procédé selon l'une quelconque des revendications précédentes, dans lequel l'activité de liaison cellulaire modifiée permet à la particule virale enveloppée de se lier à une cellule cible.

12. Procédé selon la revendication 11, dans lequel la cellule cible est une cellule de mammifère.

**13.** Procédé selon la revendication 11, dans lequel la cellule cible est une cellule humaine.

**14.** Procédé selon la revendication 11, dans lequel la cellule cible est une cellule quiescente.

**15.** Procédé selon la revendication 11, dans lequel la cellule cible est une cellule-souche hématopoïétique humaine.

**16.** Procédé selon la revendication 11, dans lequel la cellule cible est une cellule cancéreuse.

**17.** Procédé selon la revendication 11, dans lequel la cellule cible est un lymphocyte T de mammifère.

**18.** Particule virale enveloppée présentant une activité de liaison cellulaire modifiée pouvant être obtenue par un procédé selon l'une quelconque des revendications précédentes, l'activité de liaison cellulaire modifiée étant conférée par un peptide non viral différent d'un polypeptide viral chimère formé par la fusion de la partie env native du peptide viral avec un fragment peptidique de liaison, et dans laquelle l'activité de liaison cellulaire modifiée rend possible l'interaction de la particule virale avec un ou plusieurs types cellulaires différents de ceux de la particule virale non modifiée.

**19.** Particule virale enveloppée présentant une
activité de liaison cellulaire modifiée obtenue par un procédé selon l'une quelconque des revendications 1 à 17, dans laquelle l'activité de liaison cellulaire modifiée rend possible l'interaction de la particule virale avec un ou plusieurs types cellulaires différents de ceux de la particule virale non modifiée.

**20.** Procédé de préparation d'une population enrichie en un type de cellule cible à partir d'une population plus vaste de cellules, dans lequel :

(1) des particules virales enveloppées selon l'une quelconque des revendications 18 et 19, présentant une activité de liaison modifiée pour les cellules cibles, sont exposées à une population de cellules comprenant le type de cellule cible pour permettre la liaison aux particules virales ; (2) les particules virales enveloppées liées aux cellules cibles sont ensuite séparées de la population de cellules ; (3) éventuellement, les particules virales enveloppées sont ensuite retirées des cellules cibles.

**21.** Procédé d'enrichissement du titre de particules virales enveloppées incorporant un fragment peptidique de liaison passager à partir d'une population de particules virales enveloppées selon la revendication 18 ou 19, comprenant :

i) l'obtention d'un support sur lequel le fragment peptidique de liaison passager se lie ; et
ii) l'exposition de la population de particules virales enveloppées au support ; et, éventuellement
iii) l'isolement des particules virales enveloppées qui se lient au support par rapport aux particules virales qui ne se lient pas au support.

**22.** Préparation de particules virales enveloppées selon la revendication 18 ou 19 enrichie en particules virales enveloppées incorporant un fragment peptidique de liaison passager, la préparation présentant un titre en particules virales enveloppées d'au moins $10^5$ ifu/ml (unités formant infection/ml).

**23.** Préparation selon la revendication 22, comprenant en outre un excipient et/ou support pharmaceutiquement acceptable.

**24.** Particule virale enveloppée selon la revendication 18 ou 19 ou préparation de particules virales enveloppées selon la revendication 22 ou 23, pour utilisation en médecine.

**25.** Utilisation d'une particule virale enveloppée selon la revendication 18 ou 19 ou d'une préparation de particules virales enveloppées selon la revendication 22 ou 23, dans la préparation d'un médicament destiné au diagnostic et/ou à la prévention et/ou au traitement d'une maladie ou d'un trouble.

**26.** Utilisation d'une particule virale enveloppée selon la revendication 18 ou 19 ou d'une préparation de particules virales enveloppées selon la revendication 22 ou 23, dans la préparation d'un médicament destiné à la prévention et/ou au traitement de l'arthrite.

**27.** Utilisation selon la revendication 26, dans laquelle la particule virale enveloppée incorpore une molécule de liaison qui se lie à CD5 en tant que fragment peptidique de liaison passager.

28. Utilisation selon la revendication 26, dans laquelle la particule virale enveloppée incorpore un facteur de cellule-souche lié à la membrane en tant que fragment peptidique de liaison passager.

29. Utilisation d'une particule virale enveloppée selon la revendication 18 ou 19 ou d'une préparation de particules virales enveloppées selon la revendication 22 ou 23, dans la préparation d'un médicament destiné au diagnostic et/ou à la prévention et/ou au traitement d'un cancer.

30. Utilisation selon la revendication 29, dans laquelle le cancer est un cancer des ovaires.

31. Utilisation selon la revendication 29 ou 30, dans laquelle la particule virale enveloppée incorpore un facteur de cellule-souche lié à la membrane en tant que fragment peptidique de liaison passager.

32. Utilisation selon l'une quelconque des revendications 29 à 31, dans laquelle la particule virale enveloppée comprend un gène codant un polypeptide OPCML (de type protéine de liaison aux opioïdes/molécule d'adhérence cellulaire).

33. Particule virale enveloppée selon la revendication 18 ou 19 ou préparation de particules virales enveloppées selon la revendication 22 ou 23, pour utilisation dans le transfert de gène.

34. Utilisation des particules virales enveloppées selon la revendication 18 ou 19 ou d'une préparation de particules virales enveloppées selon la revendication 22 ou 23, dans la préparation d'un vaccin.

35. Composition pharmaceutique comprenant une particule virale enveloppée selon l'une quelconque des revendications 18 ou 19 ou une préparation de particules virales enveloppées selon la revendication 22 ou 23, et un support pharmaceutiquement acceptable.

# New Targeting Strategy

Engineered retroviral particles
containing mb-SCF proteins

pRep8mb-SCF

CaPO₄ Transfection

mb-SCF

Gag-pol  env

Gag-pol  env  pRep8mb-SCF

PINCO Vector

Histidinol
selection

Phoenix ecotropic
packaging cell line

Phoenix ecotropic packaging
cell line expressing mb-SCF

## Fig. 1

*Fig. 2*

Lane          1   2   3   4

913 bp

829 bp

350 bp

## Fig. 3

**Fig. 4**

EP 1 521 839 B1

*Fig. 5*

*Fig. 6*

Comparison of retrovirus titres
on NIH 3T3 cells

*Fig. 7*

Lane        1        2        3

78 kDa

44 kDa

23 kDa

*Fig. 8*

## Virus Binding Assays

*Fig. 9*

**Proliferation of Mo7e cells**

Legend:
- ◆ SCF (100 ng/ml)
- ■ Cl3P (10ul)
- ▲ Cl3P(30ul)
- ✕ Cl3PT (10ul)
- ✱ Cl3PT (30ul)
- ● No Cytokines/virus
- + EcoP (10ul)

Y-axis: Absorbance (0 to 1)

X-axis: Days (1 to 7)

*Fig. 10 (Part 1 of 2)*

Growth curve of Mo7e (blocked by anti ckit antibody)

*Fig. 10 (Part 2 of 2)*

Relative retroviral transduction efficiencies in
Mo7e cells by supernatant infection (MOI=5)

>95% kit+

*Fig. 11*

Relative retroviral transduction efficiencies in
TF-1 cells by supernatant infection (MOI=5)

>95% kit+

*Fig. 12*

**Relative retroviral transduction efficiencies in LAMA cells by supernatant infection (MOI=5)**

*Fig. 13*

**Relative retroviral transduction efficiency on U937 cells**

Legend:
- ☑ Amphotropic virus
- ☒ Ecotropic virus
- ☒ CI3 virus
- ⊞ Ampho mb-SCF virus

Retroviral supernatant (n=2 exp)

>99% kit -ve

*Fig. 14*

Fig. 15

**Relative retroviral transduction efficiency on human bone marrow MNC from B6 mice**

*Fig. 16*

Relative targeted transduction efficiencies of human bone marrow derived CFU-GM

*Fig. 17*

# Apical (outer) surface of plasma membrane

*Fig. 18*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9200376 A
- WO 9712049 A
- WO 8807378 A, Bagshawe
- WO 9111201 A, Epenetos & Rowlinson-Busza
- US 4675187 A
- US 5180818 A
- US 5168053 A
- US 5149796 A
- US 5116742 A
- US 5093246 A
- US 4987071 A
- WO 9410323 A
- WO 0172336 A

**Non-patent literature cited in the description**

- **Gollan ; Green.** *J. Virology,* 2002, 3558-3563
- **Cosset et al.** *J Virol,* 1995, vol. 69, 7430-7436
- **Sambrook et al.** MOLECULAR CLONING: A LABORATORY MANUAL. 2001
- **H Zola.** Monoclonal Antibodies: A manual of techniques. CRC Press, 1988
- **J G R Hurrell.** Monoclonal Hybridoma Antibodies: Techniques and Applications. CRC Press, 1982
- **Morrison et al.** *Proc. Natl. Acad. Sci. USA,* 1984, vol. 81, 6851-6855
- **Better et al.** *Science,* 1988, vol. 240, 1041
- **Skerra et al.** *Science,* 1988, vol. 240, 1038
- **Bird et al.** *Science,* 1988, vol. 242, 423
- **Huston et al.** *Proc. Natl. Acad. Sci. USA,* 1988, vol. 85, 5879
- **Ward et al.** *Nature,* 1989, vol. 341, 544
- **Winter ; Milstein.** *Nature,* 1991, vol. 349, 293-299
- **Searle et al.** *Br. J. Cancer,* 1981, vol. 44, 137-144
- **Senter et al.** *PNAS USA,* 1988, vol. 85, 4842-4846
- **Hellström et al.** *Cancer Res.,* 1986, vol. 46, 3917-3923
- **Clarke et al.** *Proc. Natl. Acad. Sci. USA,* 1985, vol. 82, 1766-1770
- **Beradi et al.** *Science,* 1995, vol. 267, 104-108
- **Lamb et al.** *Eur. J. Biochem.,* 1985, vol. 148, 265-270
- **Mullen et al.** *PNAS,* 1922, vol. 89, 33
- **Moolten.** *Cancer Res.,* 1986, vol. 46, 5276
- **Ezzedine et al.** *New Biol,* 1991, vol. 3, 608
- **R J Massey.** *Nature,* 1987, vol. 328, 457-458
- **Saiki et al.** *Science,* 1988, vol. 239, 487-491
- **Cohen et al.** *Proc. Natl. Acad. Sci. USA,* 1972, vol. 69, 2110
- **Sambrook et al.** Molecular Cloning, A Laboratory Manual. Cold Spring Harbor Laboratory, 1989
- **Sherman et al.** Methods In Yeast Genetics, A Laboratory Manual. Cold Spring Harbor, 1986
- **Beggs.** *Nature,* 1978, vol. 275, 104-109
- **Luchansky et al.** *Mol. Microbiol.,* 1988, vol. 2, 637-646
- **Becker ; Guarente.** *Methods Enzymol.,* 1990, vol. 194, 182
- **Southern.** *J. Mol. Biol.,* 1975, vol. 98, 503
- **Berent et al.** *Biotech.,* 1985, vol. 3, 208
- **Clewell ; Helinski.** *Biochemistry,* 1970, vol. 9, 4428-4440
- **Clewell.** *J. Bacteriol.,* 1972, vol. 110, 667-676
- **Sellar et al.** OPCML at 11q25 is epigenetically inactivated and has tumor-suppressor function in epithelial ovarian cancer. *Nat Genet.,* 2003, vol. 34 (3), 337-43
- **Lee DM ; Friend DS ; Gurish MF ; Benoist C ; Mathis D ; Brenner MB.** Mast cells: a cellular link between autoantibodies and inflammatory arthritis. *Science,* 06 September 2002, vol. 297 (5587), 1689-92
- **Verhoeyen et al.** *Immunology,* 1993, vol. 78, 364-370
- **Kabat et al.** Sequences of Proteins of Immunological Interest. US Dept of Health and Human Services Public Health Service, 1987
- Monoclonal antibodies - applications in clinical oncology. Chapman & Hall, 1991, 37-43
- Remington: The Science and Practise of Pharmacy
- **Cleland.** *Pharm. Biotechnol.,* 1997, vol. 10, 1-43
- **Lee.** *Curr. Opin. Biotechnol.,* 2001, vol. 11, 81-84

- **Cleland et al.** *J. Control. Release,* 2001, vol. 72, 13-24
- **Takeuchi et al.** *Adv. Drug. Delic. Rev.,* 2001, vol. 47, 39-54
- **Ansel.** Pharmaceutical Dosage Forms and Drug Delivery Systems. Lippincott Williams and Wilkins, 1999
- **Ager et al.** *Hum Gene Ther,* 1996, vol. 7, 2157-2164
- **Bagnis et al.** *Hum Gene Ther,* 1994, vol. 5, 1325-1333
- **Bodem et al.** *Stem Cells,* 1997, vol. 15 (1), 141-147
- **Cadd et al.** *Bioessays,* 1997, vol. 19, 993-1000
- **Cosman et al.** *Behring Inst Mitt,* 1988, 149-157
- **Cosset ; Russell.** *Gene Therapy,* 1996, vol. 3, 946-956
- **Cosset et al.** *J Virol,* 1995, vol. 69, 6314-6322
- **Cosset et al.** *J Viro/6,* 1995, vol. 9, 7430-7436
- **Dimmock ; Primrose.** Introduction to Modem Virology. Blackwell Scientific Publications, 1987
- **Etienne - Juan et al.** *J Gen Virol,* 1992, vol. 73, 3251-3255
- **Garoff et al.** *Mirobiol Mol Biol Rev,* 1998, vol. 62, 1171-1190
- **Goud et al.** *Virology,* 1998, vol. 163, 251-254
- **Griffiths ; Rottier.** *Semin Cell Biol,* 1992, vol. 3, 367-381
- **Grignangi et al.** *Cancer Res,* 1998, vol. 58, 14-19
- **Hamel ; Westphal.** *J Neuro - Oncology,* 1997, vol. 35, 327-333
- **Han et al.** *Proc Natl Acad Sci USA,* 1995, vol. 92, 9747-9751
- **Hatziioannou et al.** *J Virol,* 1998, vol. 72, 5313-5317
- **Hatziioannou et al.** *Hum Gene Ther,* 1999, vol. 10, 1533-1544
- **Hurt et al.** *J Biol Chem,* 2000, vol. 275, 35424-35431
- **Kasahara et al.** *Science,* 1994, vol. 266, 1373-1376
- **Kinsella ; Nolan.** *Hum Gene Ther,* 1996, vol. 7, 1405-1413
- **Kitamura et al.** *Cell Physiol,* 1989, vol. 140, 323-334
- **Lee et al.** *Biochem Biophys Res Commun,* 2001, vol. 280, 675-683
- **Lee et al.** *Science,* 2002, vol. 297 (5587), 1689-92
- **Lyman et al.** *Oncogene,* 1995, vol. 10, 149-157
- **Marin et al.** *J Virol,* 1996, vol. 70, 2957-2962
- **Matano et al.** *J Gen Virol,* vol. 76, 3165-3169
- **Miyazawa et al.** *Blood,* 1995, vol. 85, 641-649
- **Morgenstern ; Land.** *Nucleic Acid Res,* 1990, vol. 18, 3587-3596
- **Neda et al.** *J Biol Chem,* 1991, vol. 226, 14143-14149
- **Pickard et al.** *J Neurosci,* 2000, vol. 20, 7922-7931
- **Roux et al.** *Proc Natl Acad Sci USA,* 1989, vol. 86, 9079-9083
- **Russell et al.** *Nucl Acid Res,* 1993, vol. 21, 1081-1085
- **Russell et al.** *Nucleic Acid Res,* 1993, vol. 21, 1081-1085
- **Sambrook et al.** MOLECULAR CLONING: A LABORATORY MANUAL. Cold Spring Harbor Laboratory, 2001
- **Schnierle et al.** *Gene Therapy,* 1996, vol. 3, 334-342
- **Sehgal et al.** *Gene Ther,* 1999, vol. 6, 1084-1091
- **Seigneurin et al.** *Exp Hematol,* 1987, vol. 15, 822-832
- **Sellar et al.** *Nat Genet.,* 2003, vol. 34 (3), 337-43
- **Somia et al.** *Proc Natl Acad Sci USA,* 1995, vol. 92, 7570-7574
- **Strauss et al.** *Trends Microbiol,* 1995, vol. 3, 346-350
- **Thalmeier et al.** *Blood,* 1994, vol. 83, 1799-1807
- **Valsesia - Wittmann et al.** *J Virol,* 1994, vol. 68, 4609-4619
- **Valsesia - Wittmann et al.** *J Virol,* 1996, vol. 70, 2059-2064